# EUROPEAN PATENT APPLICATION

(11) **EP 4 682 144 A1**
(43) Date of publication of application: **21.01.2026**
(21) Application number: 24769964.8
(22) Date of filing: 13.03.2024
(51) Int. Cl.: C07D 401/14, C07D 405/14, A61K 31/4155, A61P 3/10

(54) **SOLID FORM OF PYRROLIDONE DERIVATIVE AS GLUCOKINASE ACTIVATOR**

(30) Priority: 13.03.2023 CN 202310245157
(71) Applicant: Hua Medicine (Shanghai) Ltd., Shanghai 201203 (CN)
(72) Inventor: SHE, Jin, Shanghai 201203 (CN); JIN, Xiangle, Shanghai 201203 (CN); ZHAO, Qiang, Shanghai 201203 (CN); HU, Bin, Shanghai 201203 (CN); CHEN, Li, Shanghai 201203 (CN)
(74) Representative: Cabinet Becker et Associés
(86) International application number: PCT/CN2024/081449
(87) International publication number: WO 2024/188273

(57) **Abstract**

The present invention relates to a crystal form of a free state of (R)-3-(3-((S)-2-(4-(2-chlorophenoxy)-2-oxo-2,5-dihydro-1H-pyrrol-1-yl)-4-methylpentanoylamino)- 1H-pyrazol-1-yl)-2-hydroxypropylnicotinate (compound A) or a pharmaceutically acceptable salt thereof, a pharmaceutical composition and a preparation method thereof, and a use of the crystal form in the preparation of a medicament for treating diabetes and related symptoms.

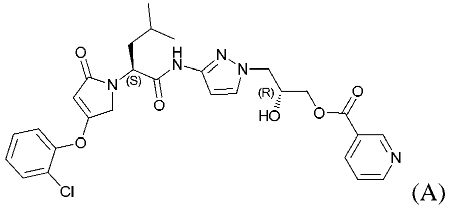

## Description

This application claims the priority of:

CN202310245157.X, filing date: March 13, 2023.

### FIELD OF THE INVENTION

The present disclosure belongs to the field of pharmaceutical technology, and specifically relates to a solid form of pyrrolidone derivatives as glucokinase activator (GKA), particularly relates to a crystal form of the free state of (R)-3-(3-((S)-2-(4-(2-chlorophenoxy)-2-oxo-2,5-dihydro-1H-pyrrol-1-yl)-4-methylpentanamido)-1H-pyrazol-1-yl)-2-hydroxypropyl nicotinate (compound A) or a pharmaceutically acceptable salt thereof, a pharmaceutical composition and a preparation method thereof, and use of the crystal form and pharmaceutical composition in the manufacture of a medicament for the treatment of diabetes mellitus and related symptoms.

### BACKGROUND OF THE INVENTION

Diabetes mellitus has become a prevalent disease worldwide. According to the data from the 24th National Conference of the Chinese Diabetes Society (CDS), China has the largest number of patients with diabetes, which in adults is about 129.8 million, over the world. Type II diabetes, i.e. non-insulin dependent diabetes mellitus (NIDDM), which comprises of more than 90% of patients with diabetes, is a hyperglycemic chronic, metabolic dysfunction resulting from an imbalance of blood glucose homeostasis in human body caused by insulin secretion disorder and insulin resistance.

Glucokinase (GK) plays a central role in stabilizing blood glucose homeostasis in human body. GK, which acts as a glucose sensor in glucose homeostasis, senses blood glucose changes, regulates the secretion of messenger glucose-controlling hormones, insulin, glucagon, and GLP-1, and constitutes a sensing system for regulation of blood glucose homeostasis in human body. GK is mainly distributed in the liver, where it rapidly converts glucose into hepatic glycogen for storage in response to elevated blood glucose, while lowering the level of glucose in the blood. Glucose reserve during glucose ingestion and glucose supply during fasting, controlled by glucose-controlling hormones, constitute the regulation of blood glucose homeostasis in human body.

Impaired function and expression of glucokinase, and the dysfunction of the sensor, result in the dysfunction of the early phase secretion of glucose-controlling hormones, affecting glucose uptake and output, and resulting in post-prandial hyperglycemia and pre-prandial hypoglycemia. Abnormal signaling of glucose-controlling hormones cause abnormal functions and expressions of key proteins in the execution system of glucose uptake and output, forming abnormal operating state, and leading to type II diabetes.

Glucokinase activators were developed against the characteristics of GK as a target, which can improve the secretion function of insulin, glucagon and GLP-1 for glucose regulation by increasing the sensitivity of α, β and L cells to changes in glucose concentration.

Compound A is a new glucokinase activator developed by the applicant of the present disclosure, with a chemical name of (R)-3-(3-((S)-2-(4-(2-chlorophenoxy)-2-oxo-2,5-dihydro-1H-pyrrol-1-yl)-4-methylpentanamido)-1H-pyrazol-1-yl)-2-hydroxypropyl nicotinate (compound A), a chemical formula of C₂₈H₃₀ClN₅O₆, and a chemical structure of:

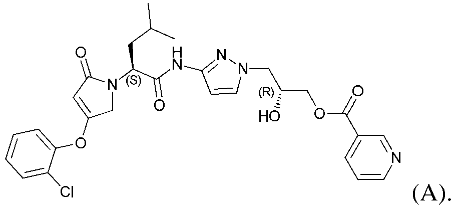

Compound A is a derivative of HMS5552, which can be efficiently converted (e.g., undergoing enzymatic and/or chemical conversion) into HMS5552 in the body (particularly in the small intestine), and thus absorbed into the circulatory system to achieve the purpose of treating or preventing some metabolic syndrome diseases. Alternatively, the compound disclosed herein is characterized in that it is stable in gastric fluid, and can be efficiently converted into HMS5552 in intestinal and small intestinal cells.

Therefore, there is a need in this field to develop a solid form (particularly a crystal form) of the free state of compound A and a salt thereof, to meet the requirements for drug properties such as activity, drug stability, and bioavailability during the manufacture, storage, and use of a medicament.

### SUMMARY OF THE INVENTION

In one aspect, the present disclosure provides a crystal form of the free state of compound A.

In one embodiment, the present disclosure provides crystal form A of the free state of compound A.

In another aspect, the present disclosure provides a crystal form of a salt of compound A. In one embodiment, the present disclosure provides a crystal form of an inorganic acid salt of compound A; in another embodiment, the present disclosure provides a crystal form of an organic acid salt of compound A.

In one specific embodiment, the present disclosure provides crystal form A of a hydrochloride of compound A; in another specific embodiment, the present disclosure provides crystal form B of a hydrochloride of compound A; in another specific embodiment, the present disclosure provides crystal form C of a hydrochloride of compound A.

In another specific embodiment, the present disclosure provides crystal form A of a maleate of compound A; in another specific embodiment, the present disclosure provides crystal form B of a maleate of compound A; in another specific embodiment, the present disclosure provides crystal form A of a naphthalene disulfonate of compound A; in another specific embodiment, the present disclosure provides crystal form A of an oxalate of compound A; in another specific embodiment, the present disclosure provides crystal form A of a hydrobromide of compound A; in another specific embodiment, the present disclosure provides crystal form B of a hydrobromide of compound A.

In another aspect, the present disclosure provides a pharmaceutical composition, comprising a crystal form of the present disclosure and one or more pharmaceutically acceptable excipients.

In another aspect, the present disclosure provides use of a crystal form of the present disclosure in the manufacture of a medicament for the treatment and/or prevention of one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

In another aspect, the present disclosure provides a crystal form of the present disclosure, for use in the treatment and/or prevention of one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

In another aspect, the present disclosure provides a method of treating and/or preventing a disease in a subject, comprising administering to the subject a crystal form of the present disclosure, wherein the disease is selected from one or more of type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

In another aspect, the present disclosure provides use of a crystal form or a pharmaceutical composition of the present disclosure in the manufacture of a medicament for curing diabetes, causing diabetes remission and/or regressing diabetes.

In another aspect, the present disclosure provides a crystal form or a pharmaceutical composition of the present disclosure, for use in curing diabetes, causing diabetes remission and/or regressing diabetes.

In another aspect, the present disclosure provides a method of curing diabetes, causing diabetes remission and/or regressing diabetes in a subject, comprising administering to the subject a crystal form or a pharmaceutical composition of the present disclosure.

### BRIEF DESCRIPTION OF THE DRAWINGS

Fig. 1 is the XRPD pattern of the starting material sample.
Fig. 2 is the TGA/mDSC curve of the starting material sample.
Fig. 3 is a graph showing the comparison of retention rate/generation rate-incubation time of compound A and HMS5552 in artificial simulated gastric fluid.
Fig. 4 is a graph showing the comparison of retention rate/generation rate-incubation time of compound A and HMS5552 in artificial simulated intestinal fluid.
Fig. 5 is a graph showing the comparison of OGTT blood glucose-time curves and AUC₀₋₁₂₀ₘᵢₙ of HMS5552 and compound A in C57BL/6J mice. In the graph, * means P<0.05 and ** means P<0.01, as compared to the Vehicle control group; and # means P<0.05 and ## means P<0.01, as compared to the corresponding HMS5552 dosing group.
Fig. 6 is a graph showing the comparison of insulin levels (0 min and 15 min) of HMS5552 and compound A in C57BL/6J mice. In the graph, * means P<0.05 and ** means P<0.01, as compared to the Vehicle control group; and # means P<0.05 and ## means P<0.01, as compared to the corresponding HMS5552 dosing group.
Fig. 7 is the XRPD pattern of crystal form A of the free state.
Fig. 8 is the TGA/DSC curve of crystal form A of the free state.
Fig. 9 is the XRPD pattern of crystal form A of the hydrochloride.
Fig. 10 is the TGA/DSC curve of crystal form A of the hydrochloride.
Fig. 11 is the XRPD pattern of crystal form A of the maleate.
Fig. 12 is the XRPD pattern of crystal form B of the maleate.
Fig. 13 is the TGA/DSC curve of crystal form B of the maleate.
Fig. 14 is the XRPD pattern of crystal form A of the naphthalene disulfonate.
Fig. 15 is the TGA/DSC curve of crystal form A of the naphthalene disulfonate.
Fig. 16 is the XRPD pattern of crystal form A of the oxalate.
Fig. 17 is the TGA/DSC curve of crystal form A of the oxalate.
Fig. 18 is the XRPD pattern of crystal form A of the hydrobromide.
Fig. 19 is the XRPD pattern of crystal form B of the hydrobromide.
Fig. 20 is the TGA/DSC curve of crystal form B of the hydrobromide.
Fig. 21 shows the overlaid XRPD pattern of crystal form A of the free state prepared by solution crystallization.
Fig. 22 is the XRPD pattern of crystal form B of the hydrochloride.
Fig. 23 is the TGA/DSC curve of crystal form B of the hydrochloride.
Fig. 24 is the XRPD pattern of crystal form C of the hydrochloride.
Fig. 25 is the TGA/DSC curve of crystal form C of the hydrochloride.
Fig. 26 and Fig. 27 show DVS tests of crystal form A of the free state and crystal form B of the hydrochloride.
Fig. 28 and Fig. 29 are graphs showing the comparison of XRPD patterns of crystal form A of the free state before and after dissolution stability tests in media with different pHs and biologically relevant media.
Figs. 30-33 are graphs showing the comparison of XRPD patterns of crystal form A of the free state before and after the forced degradation stability test.

### DETAILED DESCRIPTION OF THE INVENTION

### Definitions

Unless otherwise specified, all technical and scientific terms used herein have the same meanings as commonly understood by those skilled in the art to which the present disclosure belongs, but in case of conflict, the definitions in this specification shall prevail.

As used in the specification and claims, the singular forms "a", "an" and "the (said)" include plural forms, unless clearly specified otherwise in the context.

All numerical values or expressions used in the specification and claims should be understood to be modified by "about" in all cases. The term "about" when referring to an amount or a numerical range means that the amount or the numerical range referred to is an approximate value within experimental variability (or within statistical experimental error). Therefore, the amount or the numerical range can be varied between, for example, ±10%, alternatively ±5% of the amount or the numerical range referred to.

As used herein, the term "substantially" refers to taking the typical variability of a particular method and the standard error of a measured value into account. For example, with respect to the position of an X-ray powder diffraction peak, the term "substantially" refers to taking the typical variability of the position and intensity of a peak into account. Those skilled in the art will recognize that the peak position (2θ) may exhibit some variabilities, typically up to ±0.2°. Additionally, those skilled in the art will recognize that the relative peak intensities may exhibit equipment-to-equipment variability as well as variabilities due to crystallinity, preferred orientation, tested sample surface, and other factors known to those skilled in the art. Similarly, tests such as Differential Scanning Calorimetry (DSC) and Thermogravimetric Analysis (TGA) may also exhibit variabilities caused by equipment-to-equipment variability, as well as crystallinity, preferred orientation, tested sample surface, and other factors known to those skilled in the art. Usually, the error range of DSC is ±5 °C, alternatively ±4 °C, alternatively ±3 °C, alternatively ±2 °C, alternatively ±1 °C, or alternatively ±0.5 °C; the error range of TGA is ±2.5%, alternatively ±2%, alternatively ±1.5%, alternatively ±1%, or alternatively ±0.5%.

As used herein, the terms "crystalline" and "crystal form" refer to a solid composed of molecules with a regular repeating arrangement. Crystalline forms may differ in thermodynamic stability, physical parameters, X-ray structures and preparation processes.

The term "amorphous" refers to a solid composed of molecules arranged in a disordered manner.

As used herein, the term "solvate" refers to a crystal form having a stoichiometric or non-stoichiometric amount of a solvent (such as water, methanol, ethyl acetate, etc., or mixtures thereof) in the crystal lattice by non-covalent intermolecular bonding. The term "hydrate" refers to a solvate in which the solvent is water.

As used herein, the term "anhydrous" refers to a crystalline form containing less than about 1% (w/w) adsorbed water as determined by standard methods such as Karl Fisher analysis.

As used in this specification and in the claims, "and/or" should be understood to mean "either or both" of the associated elements, i.e., the elements exist jointly in some cases and separately in other cases. Multiple elements listed with "and/or" should be construed in the same fashion, i.e., "one or more" of the associated elements. In addition to the elements specifically identified by the "and/or" clause, other elements may optionally exist, whether related or unrelated to those elements specifically identified. Thus, as a non-limiting example, a reference to "A and/or B", when used in conjunction with open-ended language such as "comprising", can refer, in one embodiment, to A only (optionally including elements other than B); in another embodiment, to B only (optionally including elements other than A); in yet another embodiment, to both A and B (optionally including other elements); etc.

By "pharmaceutically available" or "pharmaceutically acceptable", it is meant a material which is not biologically or otherwise undesirable, i.e., the material may be administered to an individual without causing any undesirable biological effects or interacting in a deleterious manner with any other component of a composition comprising the material.

The term "pharmaceutically acceptable salt" refers to a salt that does not irritate an organism significantly and retains the biological activity and properties of a compound.

The term "pharmaceutically acceptable carrier" refers to an inactive ingredient that does not irritate an organism significantly and does not abrogate the biological activity and properties of an administered compound. As used herein, "carrier" and "excipient" have the same meaning.

The term "therapeutically effective amount" refers to an amount of an agent sufficient to provide a desired biological result. The result may be reduction and/or alleviation of a sign, symptom, or cause of a disease, or any other desired change of a biological system. For example, a "therapeutically effective amount" for therapeutic use refers to a necessary amount of a composition comprising a compound disclosed herein as an active ingredient for providing a clinically significant decrease in a disease. In any individual case, an appropriate "therapeutically effective amount" may be determined by one of ordinary skill in the art using routine experimentation. Thus, the expression "therapeutically effective amount" generally refers to an amount of an active substance at which it has a therapeutic effect.

As used herein, the term "treat" is synonymous with the terms "prevent" and "alleviate", and is intended to mean delaying disease progression, preventing disease progression and/or reducing the severity of symptoms that will develop or are expected to develop. Thus, these terms include ameliorating existing disease symptoms, preventing additional symptoms, ameliorating or preventing underlying metabolic causes of symptoms, inhibiting disorder or disease, e.g., preventing the development of disorder or disease, relieving disorder or disease, causing a regression of disorder or disease, relieving a condition caused by disease or disorder, or stopping symptoms of disease or disorder.

As used herein, the term "subject" encompasses mammals and non-mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one embodiment of the present disclosure, the mammal is a human. The term "subject" includes a confirmed patient, but the "subject" does not need to have any special identity to a hospital, clinic, or research facility (e.g., as a confirmed patient, study participant, etc.).

It is to be understood that the terminology employed herein is for the purpose of describing particular embodiments, but not intended to be limiting. The present disclosure may be understood more readily by reference to the following detailed description of the embodiments of the present disclosure and the examples included herein. Further, alternative methods, devices and materials are described below, although any methods, devices and materials similar or equivalent to those described herein can be used in the practice or testing of the present disclosure.

### Compound A and crystal form thereof

Compound, (R)-3-(3-((S)-2-(4-(2-chlorophenoxy)-2-oxo-2,5-dihydro-1H-pyrrol-1-yl)-4-methylpentanamido)-1H-pyrazol-1-yl)-2-hydroxypropyl nicotinate, referred to herein as compound A, or the free state of compound A, has the following formula:

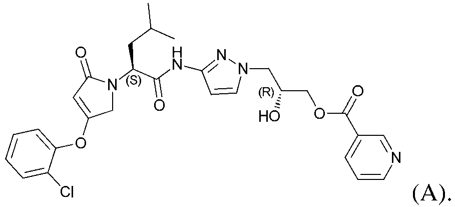

The present disclosure relates to a crystalline form of compound A, i.e., "crystal form A of compound A". In some embodiments, these crystal forms of the compound are anhydrates.

### Crystal form A of compound A

In one embodiment, the present disclosure provides crystal form A of compound A, which is an anhydrate.

In another embodiment, the X-ray powder diffraction pattern of crystal form A obtained using CuKα radiation includes at least the characteristic peaks represented by the following °2θ: 8.5±0.2, 17.7±0.2, 18.0±0.2, and 18.6±0.2. In another embodiment, the X-ray powder diffraction pattern further includes characteristic peaks represented by the following °2θ: 10.1+0.2, 14.5±0.2, 20.2±0.2, 20.6±0.2, 25.1±0.2, and 25.7± 0.2. In another embodiment, the X-ray powder diffraction pattern further includes characteristic peaks represented by the following °2θ: 12.9±0.2, 15.2±0.2, 22.0±0.2, 23.2±0.2, and 23.9±0.2. In another embodiment, the X-ray powder diffraction pattern further includes characteristic peaks represented by the following °2θ: 11.2±0.2, 12.1±0.2, 14.2±0.2, 15.7±0.2, 19.6±0.2, 21.2±0.2, 28.6±0.2, and 30.0±0.2.

In another embodiment, the X-ray powder diffraction pattern has the following characteristic peaks:

| Angle °2θ±0.2 °2θ | Relative intensity % |
|---|---|
| 8.54 | 97.6 |
| 10.05 | 36.1 |
| 11.23 | 13.8 |
| 12.10 | 20.5 |
| 12.91 | 25.9 |
| 14.19 | 28.0 |
| 14.48 | 36.3 |
| 15.25 | 21.7 |
| 15.67 | 7.4 |
| 17.72 | 97.9 |
| 18.02 | 100.0 |
| 18.55 | 78.1 |
| 19.55 | 12.9 |
| 20.17 | 37.3 |
| 20.60 | 45.3 |
| 21.24 | 5.8 |
| 22.01 | 25.1 |
| 23.16 | 19.0 |
| 23.94 | 21.6 |
| 25.09 | 30.8 |
| 25.74 | 33.7 |
| 28.62 | 9.2 |
| 29.98 | 6.6 |

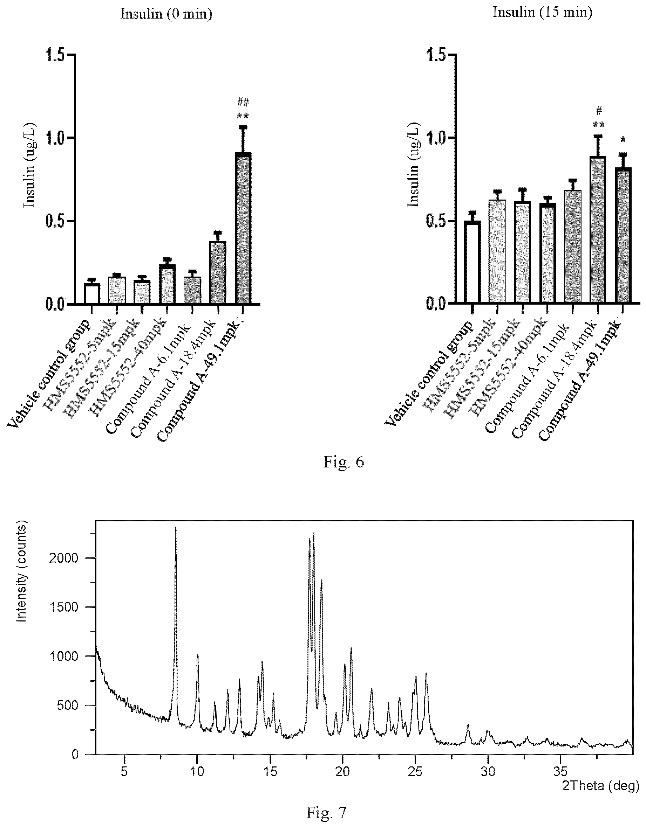

In another embodiment, the X-ray powder diffraction pattern includes one or more peaks at the 2θ in Table 18. In another embodiment, the X-ray powder diffraction pattern is substantially as shown in Fig. 7.

In another embodiment, the crystal form A has an endothermic peak at 135.5±2 °C in the differential scanning calorimetry analysis.

In another embodiment, the crystal form A has a weight loss of 1.36±0.5% at 150°C in the thermogravimetric analysis.

### Salts of compound A and crystal forms thereof

The present disclosure relates to multiple salts of compound A, such as hydrochloride, maleate, naphthalene disulfonate, oxalate, and hydrobromide.

The present disclosure also relates to crystal forms of multiple salts of compound A, such as "crystal form A of a hydrochloride of compound A", "crystal form B of a hydrochloride of compound A", "crystal form C of a hydrochloride of compound A", "crystal form A of a maleate of compound A", "crystal form B of a maleate of compound A", "crystal form A of a naphthalene disulfonate of compound A", "crystal form A of an oxalate of compound A", "crystal form A of a hydrobromide of compound A", and "crystal form B of a hydrobromide of compound A".

### Crystal form A of a hydrochloride of compound A

In one embodiment, the present disclosure provides crystal form A of a hydrochloride of compound A. In another embodiment, crystal form A of the hydrochloride is an anhydrate.

In another embodiment, the X-ray powder diffraction pattern of crystal form A of the hydrochloride obtained using CuKα radiation includes at least the characteristic peaks represented by the following °2θ: 4.1±0.2, 15.3±0.2, and 18.5+0.2. In another embodiment, the X-ray powder diffraction pattern further includes characteristic peaks represented by the following °2θ: 8.1±0.2, 10.6±0.2, 11.9±0.2, 22.1±0.2, and 22.9±0.2. In another embodiment, the X-ray powder diffraction pattern further includes characteristic peaks represented by the following °2θ: 12.2+0.2, 17.9±0.2, 21.5±0.2, and 24.9±0.2.

In another embodiment, the X-ray powder diffraction pattern has the following characteristic peaks:

| Angle °2θ±0.2 °2θ | Relative intensity % |
|---|---|
| 4.06 | 86.1 |
| 8.11 | 30.3 |
| 10.61 | 36.7 |
| 11.89 | 39.2 |
| 12.18 | 23.0 |
| 15.27 | 66.8 |
| 15.66 | 19.5 |
| 16.28 | 12.1 |
| 16.68 | 20.0 |
| 17.89 | 29.9 |
| 18.45 | 100.0 |
| 19.45 | 12.6 |
| 20.41 | 15.7 |
| 21.51 | 23.7 |
| 22.14 | 32.6 |
| 22.94 | 32.0 |
| 23.75 | 5.8 |
| 24.94 | 26.0 |
| 26.89 | 21.1 |
| 27.53 | 11.9 |
| 28.23 | 9.8 |
| 30.64 | 6.5 |

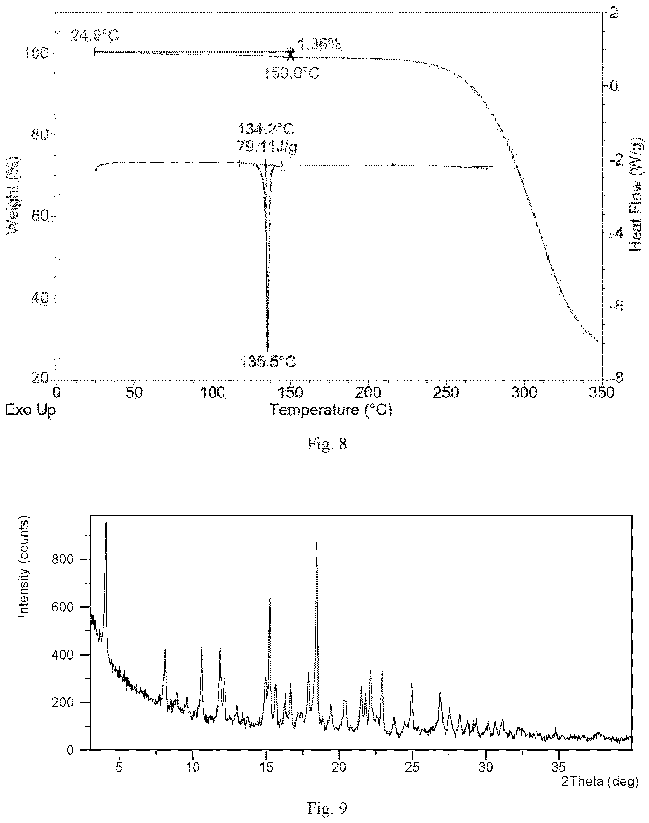

In another embodiment, the X-ray powder diffraction pattern includes one or more peaks at the 2θ in Table 21. In another embodiment, the X-ray powder diffraction pattern is substantially as shown in Fig. 9.

In another embodiment, crystal form A of the hydrochloride has endothermic peaks at 125.9±2 °C and 177.8±2 °C in the differential scanning calorimetry analysis.

In another embodiment, crystal form A of the hydrochloride has a weight loss of 4.91±0.5% at 150°C in the thermogravimetric analysis.

### Crystal form B of a hydrochloride of compound A

In one embodiment, the present disclosure provides crystal form B of a hydrochloride of compound A. In another embodiment, crystal form B of the hydrochloride is an anhydrate.

In another embodiment, the X-ray powder diffraction pattern of crystal form B of the hydrochloride obtained using CuKα radiation includes at least the characteristic peaks represented by the following °2θ: 4.5±0.2, 18.1±0.2, and 21.4±0.2. In another embodiment, the X-ray powder diffraction pattern further includes characteristic peaks represented by the following °2θ: 11.1±0.2 and 21.7±0.2. In another embodiment, the X-ray powder diffraction pattern further includes characteristic peaks represented by the following °2θ: 8.7±0.2, 13.7±0.2, 14.4±0.2, 15.1±0.2, and 23.2±0.2.

In another embodiment, the X-ray powder diffraction pattern has the following characteristic peaks:

| Angle °2θ±0.2 °2θ | Relative intensity % |
|---|---|
| 4.46 | 66.9 |
| 8.73 | 26.1 |
| 11.09 | 38.6 |
| 13.66 | 21.8 |
| 14.35 | 27.7 |
| 15.07 | 28.4 |
| 16.46 | 19.0 |
| 17.44 | 18.9 |
| 18.13 | 70.4 |
| 18.83 | 17.6 |
| 21.35 | 100.0 |
| 21.67 | 44.8 |
| 22.19 | 14.1 |
| 23.17 | 21.4 |
| 24.36 | 13.4 |
| 25.43 | 7.4 |
| 25.96 | 14.7 |
| 26.78 | 13.9 |
| 27.47 | 11.4 |
| 28.37 | 10.4 |
| 29.50 | 6.5 |
| 30.36 | 5.8 |
| 31.35 | 8.7 |
| 32.34 | 7.2 |
| 34.95 | 6.7 |

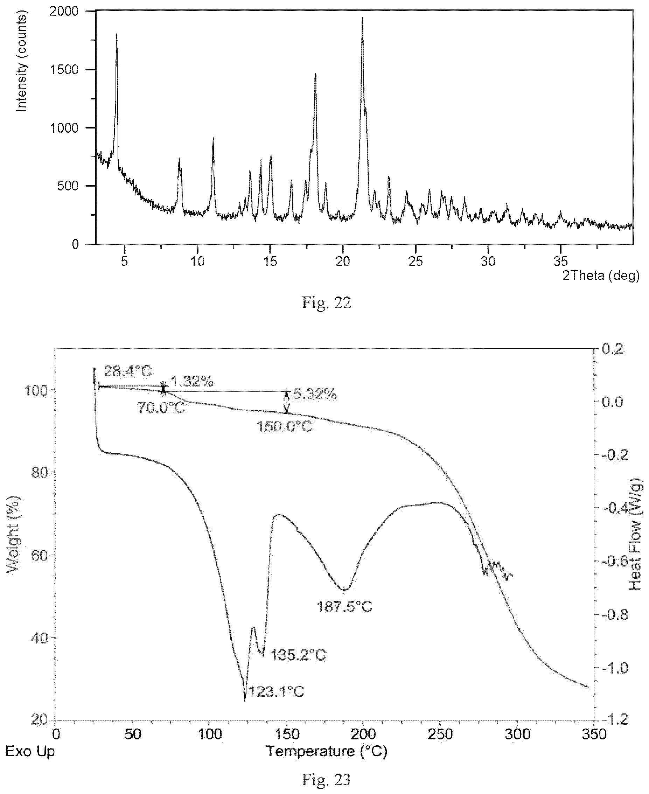

In another embodiment, the X-ray powder diffraction pattern includes one or more peaks at the 20 in Table 44. In another embodiment, the X-ray powder diffraction pattern is substantially as shown in Fig. 22.

In another embodiment, crystal form B of the hydrochloride has endothermic peaks at 123.1±2°C, 135.2±2 °C, and 187.5±2 °C in the differential scanning calorimetry analysis.

In another embodiment, crystal form B of the hydrochloride has a weight loss of 1.32±0.5% at 70 °C, and a weight loss of 5.32±0.5% at 70 to 150 °C in the thermogravimetric analysis.

### Crystal form C of a hydrochloride of compound A

In one embodiment, the present disclosure provides crystal form C of a hydrochloride of compound A. In another embodiment, crystal form C of the hydrochloride is an anhydrate.

In another embodiment, the X-ray powder diffraction pattern of crystal form C of the hydrochloride obtained using CuKα radiation includes at least the characteristic peaks represented by the following °2θ: 8.2±0.2, 10.6±0.2, 13.4±0.2, and 20.2±0.2. In another embodiment, the X-ray powder diffraction pattern further includes characteristic peaks represented by the following °2θ: 16.8±0.2 and 17.7±0.2. In another embodiment, the X-ray powder diffraction pattern further includes characteristic peaks represented by the following °2θ: 4.1±0.2, 8.8±0.2, and 12.2±0.2.

In another embodiment, the X-ray powder diffraction pattern has the following characteristic peaks:

| Angle °2θ±0.2 °2θ | Relative intensity % |
|---|---|
| 4.11 | 24.8 |
| 8.16 | 78.6 |
| 8.78 | 28.8 |
| 10.61 | 100.0 |
| 12.24 | 32.4 |
| 12.52 | 17.6 |
| 13.41 | 71.0 |
| 15.05 | 16.2 |
| 16.78 | 48.8 |
| 17.72 | 57.7 |
| 19.06 | 8.8 |
| 20.22 | 60.2 |
| 21.52 | 18.1 |
| 24.64 | 19.7 |
| 26.66 | 20.6 |
| 27.73 | 21.7 |

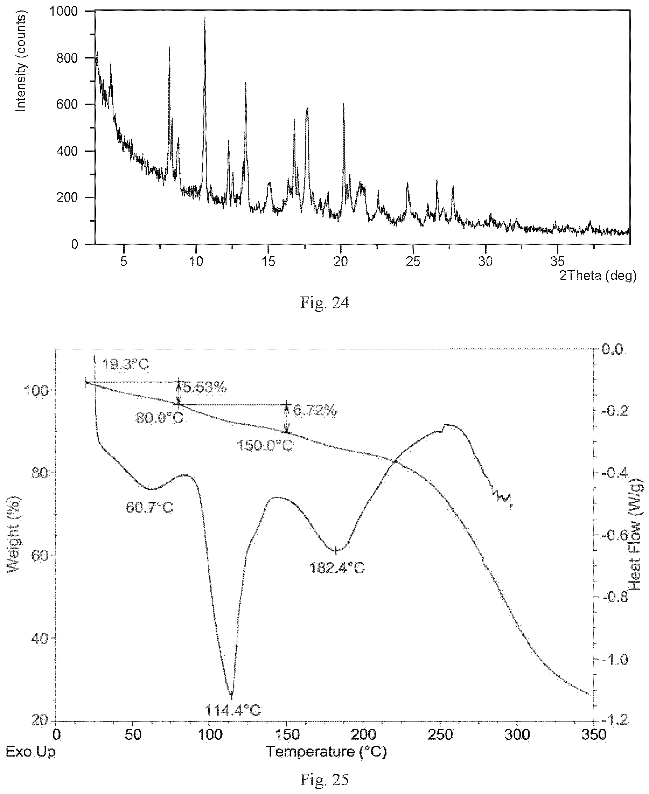

In another embodiment, the X-ray powder diffraction pattern includes one or more peaks at the 2θ in Table 45. In another embodiment, the X-ray powder diffraction pattern is substantially as shown in Fig. 24.

In another embodiment, crystal form C of the hydrochloride has endothermic peaks at 60.7±2 °C, 114.4±2°C, and 182.4±2 °C in the differential scanning calorimetry analysis.

In another embodiment, crystal form C of the hydrochloride has a weight loss of 5.53±0.5% at 80°C, and a weight loss of 6.72±0.5% at 80 to 150 °C in the thermogravimetric analysis.

### Crystal form A of a maleate of compound A

In one embodiment, the present disclosure provides crystal form A of a maleate of compound A. In another embodiment, crystal form A of the maleate is an anhydrate.

In another embodiment, the X-ray powder diffraction pattern of crystal form A of the maleate obtained using CuKα radiation includes at least the characteristic peaks represented by the following °2θ: 6.0±0.2 and 18.4±0.2. In another embodiment, the X-ray powder diffraction pattern further includes characteristic peaks represented by the following °2θ: 10.4±0.2, 15.9±0.2, and 17.4±0.2. In another embodiment, the X-ray powder diffraction pattern further includes characteristic peaks represented by the following °2θ: 6.9±0.2, 12.0±0.2, 21.2±0.2, and 21.8±0.2.

In another embodiment, the X-ray powder diffraction pattern has the following characteristic peaks:

| Angle °2θ±0.2 °2θ | Relative intensity % |
|---|---|
| 6.01 | 100.0 |
| 6.92 | 12.6 |
| 10.41 | 18.6 |
| 12.01 | 12.2 |
| 15.93 | 17.4 |
| 17.38 | 30.8 |
| 18.42 | 57.6 |
| 19.46 | 8.1 |
| 21.21 | 12.1 |
| 21.76 | 14.4 |
| 22.93 | 5.1 |

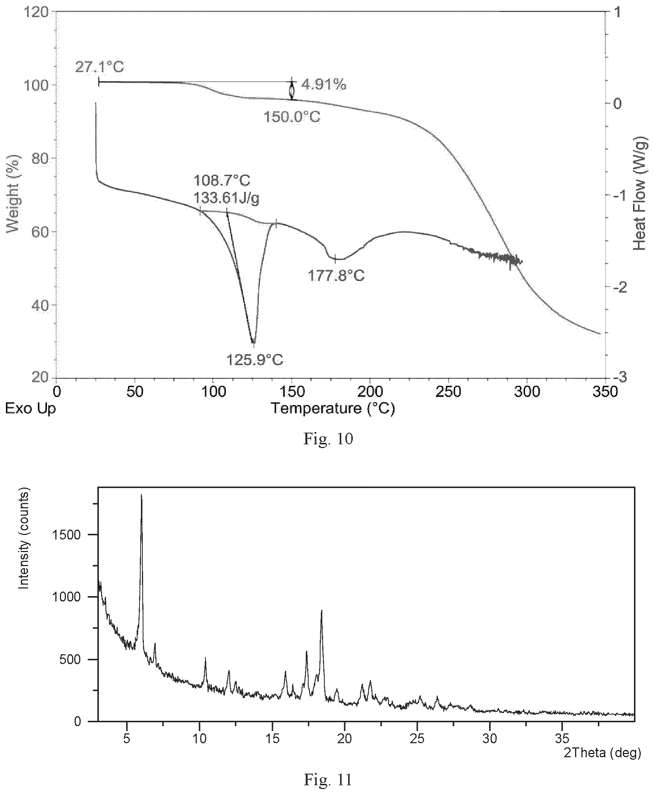

In another embodiment, the X-ray powder diffraction pattern includes one or more peaks at the 2θ in Table 22. In another embodiment, the X-ray powder diffraction pattern is substantially as shown in Fig. 11.

### Crystal form B of a maleate of compound A

In one embodiment, the present disclosure provides crystal form B of a maleate of compound A. In another embodiment, crystal form B of the maleate is an anhydrate.

In another embodiment, the X-ray powder diffraction pattern of crystal form B of the maleate obtained using CuKα radiation includes at least the characteristic peaks represented by the following °2θ: 4.1±0.2, 8.1±0.2, 16.7±0.2, and 20.2±0.2. In another embodiment, the X-ray powder diffraction pattern further includes characteristic peaks represented by the following °2θ: 17.5±0.2 and 21.3±0.2. In another embodiment, the X-ray powder diffraction pattern further includes characteristic peaks represented by the following °2θ: 16.2±0.2, 20.4±0.2, 22.4±0.2, 24.1±0.2, 24.6±0.2, and 26.5± 0.2.

In another embodiment, the X-ray powder diffraction pattern has the following characteristic peaks:

| Angle °2θ±0.2 °2θ | Relative intensity % |
|---|---|
| 4.08 | 79.3 |
| 8.13 | 94.1 |
| 10.50 | 21.5 |
| 12.20 | 27.7 |
| 13.31 | 13.4 |
| 15.01 | 21.4 |
| 16.24 | 31.0 |
| 16.68 | 72.3 |
| 17.54 | 60.1 |
| 18.37 | 12.5 |
| 20.17 | 100.0 |
| 20.41 | 34.0 |
| 21.34 | 67.4 |
| 22.40 | 34.6 |
| 22.69 | 26.7 |
| 23.16 | 13.7 |
| 24.14 | 49.6 |
| 24.56 | 30.9 |
| 25.97 | 21.7 |
| 26.55 | 34.4 |
| 27.59 | 20.4 |
| 29.90 | 15.4 |

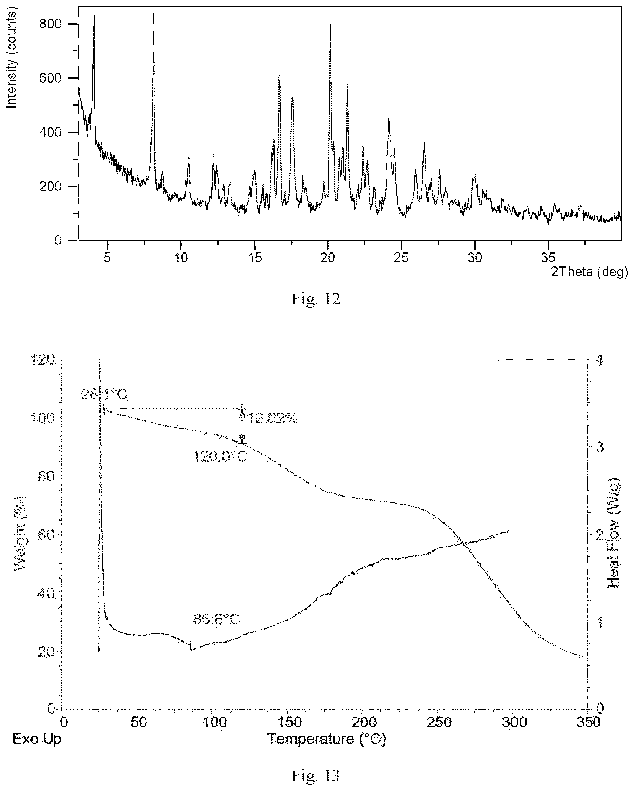

In another embodiment, the X-ray powder diffraction pattern includes one or more peaks at the 2θ in Table 23. In another embodiment, the X-ray powder diffraction pattern is substantially as shown in Fig. 12.

In another embodiment, crystal form B of the maleate has an endothermic peak at 85.6±2 °C in the differential scanning calorimetry analysis.

In another embodiment, crystal form B of the maleate has a weight loss of 12.02±0.5% at 120°C in the thermogravimetric analysis.

### Crystal form A of a naphthalene disulfonate of compound A

In one embodiment, the present disclosure provides crystal form A of a naphthalene disulfonate of compound A. In another embodiment, crystal form A of the naphthalene disulfonate is an anhydrate.

In another embodiment, the X-ray powder diffraction pattern of crystal form A of the naphthalene disulfonate obtained using CuKα radiation includes at least the characteristic peaks represented by the following °2θ: 4.0±0.2 and 13.7±0.2. In another embodiment, the X-ray powder diffraction pattern further includes characteristic peaks represented by the following °2θ: 10.3±0.2, 15.5±0.2, 21.0±0.2, and 22.3±0.2.

In another embodiment, the X-ray powder diffraction pattern has the following characteristic peaks:

| Angle °2θ±0.2 °2θ | Relative intensity % |
|---|---|
| 4.03 | 100.0 |
| 10.28 | 31.9 |
| 11.96 | 12.6 |
| 13.68 | 67.6 |
| 15.49 | 28.7 |
| 20.96 | 22.4 |
| 22.27 | 28.0 |
| 26.22 | 15.1 |

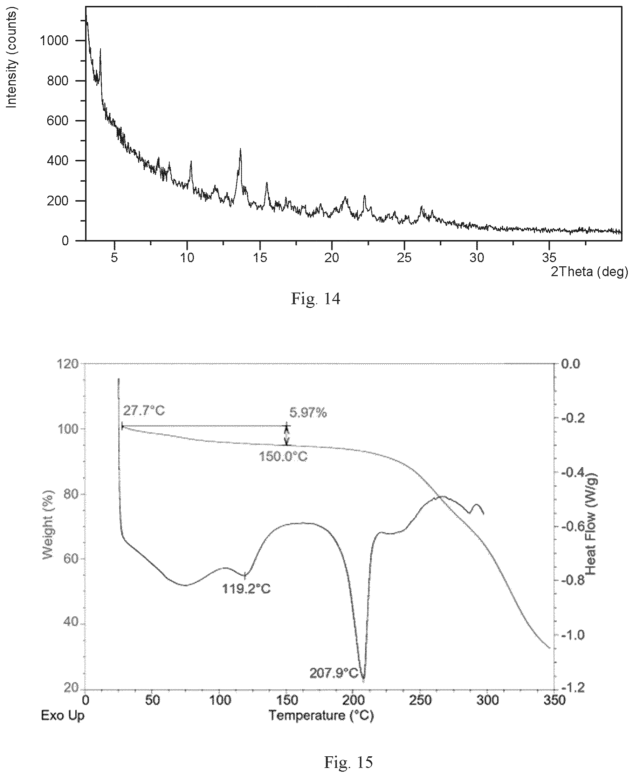

In another embodiment, the X-ray powder diffraction pattern includes one or more peaks at the 2θ in Table 24. In another embodiment, the X-ray powder diffraction pattern is substantially as shown in Fig. 14.

In another embodiment, crystal form A of the naphthalene disulfonate has endothermic peaks at 119.2±2 °C and 207.9±2 °C in the differential scanning calorimetry analysis.

In another embodiment, crystal form A of the naphthalene disulfonate has a weight loss of 5.97±0.5% at 150 °C in the thermogravimetric analysis.

### Crystal form A of an oxalate of compound A

In one embodiment, the present disclosure provides crystal form A of an oxalate of compound A. In another embodiment, crystal form A of the oxalate is an anhydrate.

In another embodiment, the X-ray powder diffraction pattern of crystal form A of the oxalate obtained using CuKα radiation includes at least the characteristic peaks represented by the following °2θ: 5.0±0.2 and 15.0±0.2. In another embodiment, the X-ray powder diffraction pattern further includes characteristic peaks represented by the following °2θ: 7.4±0.2, 16.5±0.2, 19.3±0.2, 20.1±0.2, 25.2±0.2, and 26.0±0.2.

In another embodiment, the X-ray powder diffraction pattern has the following characteristic peaks:

| Angle °2θ±0.2 °2θ | Relative intensity % |
|---|---|
| 5.00 | 100.0 |
| 7.37 | 35.6 |
| 15.05 | 60.5 |
| 16.52 | 41.7 |
| 17.39 | 12.7 |
| 18.63 | 8.7 |
| 19.31 | 27.4 |
| 20.10 | 28.4 |
| 20.68 | 13.3 |
| 22.72 | 14.7 |
| 25.21 | 25.8 |
| 26.02 | 21.2 |

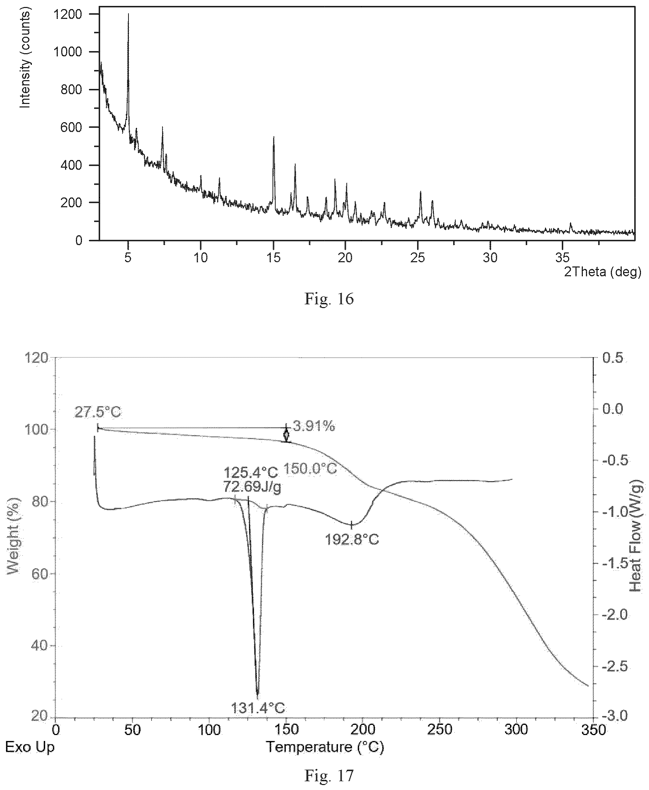

In another embodiment, the X-ray powder diffraction pattern includes one or more peaks at the 20 in Table 25. In another embodiment, the X-ray powder diffraction pattern is substantially as shown in Fig. 16.

In another embodiment, crystal form A of the oxalate has endothermic peaks at 131.4±2 °C and 192.8±2 °C in the differential scanning calorimetry analysis.

In another embodiment, crystal form A of the oxalate has a weight loss of 3.91±0.5% at 150°C in the thermogravimetric analysis.

### Crystal form A of a hydrobromide of compound A

In one embodiment, the present disclosure provides crystal form A of a hydrobromide of compound A. In another embodiment, crystal form A of the hydrobromide is an anhydrate.

In another embodiment, the X-ray powder diffraction pattern of crystal form A of the hydrobromide obtained using CuKα radiation includes at least the characteristic peaks represented by the following °2θ: 4.3±0.2 and 8.3±0.2. In another embodiment, the X-ray powder diffraction pattern further includes characteristic peaks represented by the following °2θ: 12.4±0.2, 17.5±0.2, 17.9±0.2, 20.5±0.2, and 21.3±0.2.

In another embodiment, the X-ray powder diffraction pattern has the following characteristic peaks:

| Angle °2θ±0.2 °2θ | Relative intensity % |
|---|---|
| 4.26 | 42.7 |
| 8.29 | 100.0 |
| 10.63 | 5.4 |
| 12.42 | 29.3 |
| 14.81 | 19.3 |
| 16.53 | 11.4 |
| 16.86 | 19.9 |
| 17.53 | 26.9 |
| 17.90 | 22.6 |
| 20.50 | 32.3 |
| 21.31 | 35.0 |
| 22.52 | 9.6 |
| 22.96 | 9.1 |
| 24.60 | 9.8 |
| 24.96 | 12.8 |

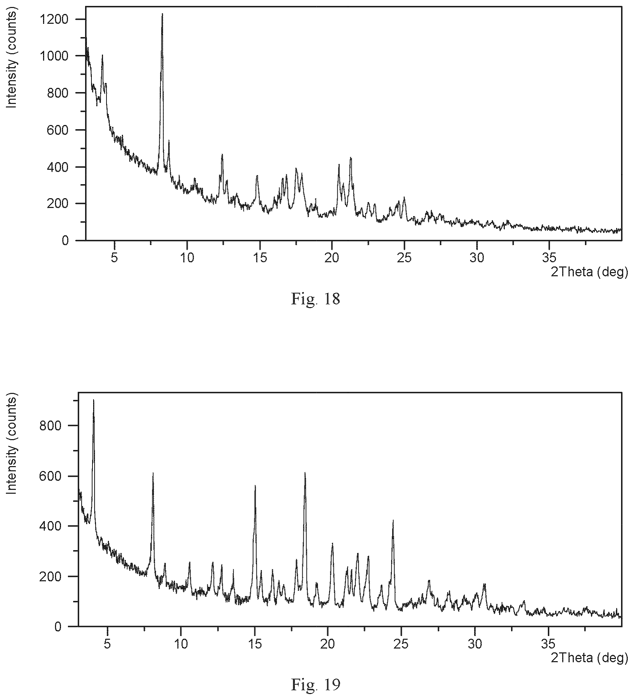

In another embodiment, the X-ray powder diffraction pattern includes one or more peaks at the 2θ in Table 26. In another embodiment, the X-ray powder diffraction pattern is substantially as shown in Fig. 18.

### Crystal form B of a hydrobromide of compound A

In one embodiment, the present disclosure provides crystal form B of a hydrobromide of compound A. In another embodiment, crystal form B of the hydrobromide is an anhydrate.

In another embodiment, the X-ray powder diffraction pattern of crystal form B of the hydrobromide obtained using CuKα radiation includes at least the characteristic peaks represented by the following °2θ: 4.1±0.2, 8.1±0.2, 15.0±0.2, 18.4±0.2, and 24.4±0.2. In another embodiment, the X-ray powder diffraction pattern further includes characteristic peaks represented by the following °2θ: 17.8±0.2, 20.3±0.2, 21.3±0.2, 22.0±0.2, and 22.8±0.2.

In another embodiment, the X-ray powder diffraction pattern has the following characteristic peaks:

| Angle °2θ±0.2 °2θ | Relative intensity % |
|---|---|
| 4.05 | 100.0 |
| 8.10 | 69.4 |
| 10.57 | 17.3 |
| 12.14 | 19.9 |
| 12.74 | 16.7 |
| 13.54 | 14.6 |
| 15.05 | 74.2 |
| 15.43 | 18.4 |
| 16.23 | 19.8 |
| 17.84 | 27.2 |
| 18.45 | 82.8 |
| 19.27 | 12.0 |
| 20.35 | 37.7 |
| 21.33 | 24.5 |
| 22.02 | 33.6 |
| 22.77 | 32.0 |
| 23.65 | 12.8 |
| 24.44 | 55.0 |
| 26.84 | 14.5 |
| 30.65 | 12.9 |

In another embodiment, the X-ray powder diffraction pattern includes one or more peaks at the 2θ in Table 27. In another embodiment, the X-ray powder diffraction pattern is substantially as shown in Fig. 19.

In another embodiment, crystal form B of the hydrobromide has endothermic peaks at 101.6±2°C and 113.3±2 °C in the differential scanning calorimetry analysis.

In another embodiment, crystal form B of the hydrobromide has a weight loss of 4.59 ± 0.5% at 90 °C in the thermogravimetric analysis.

### Drug combination and/or pharmaceutical composition

The crystal forms of the present disclosure may be used alone or in combination with other therapeutic agents to treat a variety of conditions or diseases. The crystal forms of the present disclosure and other therapeutic agents may be administered simultaneously (in the same dosage form or in separate dosage forms) or sequentially.

In one embodiment, the other therapeutic agent in combination with the crystal form of the present disclosure is a hypoglycemic agent.

In another embodiment, provided herein is a pharmaceutical composition comprising a crystal form of the present disclosure; and optionally one or more pharmaceutically acceptable excipients.

### Use for treatment and/or prevention of disease

A further embodiment of the present disclosure relates to use of a crystal form of the present disclosure or a pharmaceutical composition thereof in the manufacture of a medicament. In particular, a specific embodiment of the present disclosure relates to the use of a crystal form of the present disclosure, or a pharmaceutical composition thereof in the manufacture of a medicament for the treatment and/or prevention of the following diseases and medical conditions, in particular one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

A further specific embodiment of the present disclosure relates to the use of a crystal form of the present disclosure, or a pharmaceutical composition thereof in the manufacture of a medicament for curing diabetes, causing diabetes remission or regressing diabetes.

### Method for treatment and/or prevention of disease

A further embodiment of the present disclosure relates to a method of treating and/or preventing one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome, comprising administering to a subject a therapeutically effective amount of a crystal form of the present disclosure; or administering to the subject a therapeutically effective amount of a drug combination or a pharmaceutical composition comprising the crystal form.

The method of the present disclosure for the treatment and/or prevention of diabetes mellitus and related diseases comprises:
- preventing, slowing the progression of, delaying or treating a metabolic disorder selected from the group consisting of type I diabetes mellitus, type II diabetes mellitus, impaired glucose tolerance, impaired fasting blood glucose, hyperglycemia, postprandial hyperglycemia, hypertension, overweight, obesity, insulin resistance, and metabolic syndrome; or
- curing diabetes, causing diabetes remission or regressing diabetes; or
- improving glycemic control and/or for reducing of fasting plasma glucose, of postprandial plasma glucose and/or of glycosylated hemoglobin HbAlc; or
- preventing, slowing, delaying or reversing progression from impaired glucose tolerance, insulin resistance and/or from metabolic syndrome to type II diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, learning and memory dysfunction, neurodegenerative or cognitive disorders, cardio- or cerebrovascular diseases, tissue ischaemia, diabetic foot or ulcer, arteriosclerosis, hypertension, endothelial dysfunction, myocardial infarction, acute coronary syndrome, unstable angina pectoris, stable angina pectoris, stroke, peripheral arterial occlusive disease, cardiomyopathy, heart failure, heart rhythm disorders and vascular restenosis; or
- reducing body weight and/or body fat or preventing an increase in body weight and/or body fat or facilitating a reduction in body weight and/or body fat; or
- preventing, slowing, delaying, or treating the degeneration of pancreatic beta cells and/or the decline of the functionality of pancreatic beta cells and/or for improving and/or restoring or protecting the functionality of pancreatic beta cells and/or restoring the functionality of pancreatic insulin secretion; or
- preventing, slowing, delaying, or treating diseases or conditions attributed to an abnormal accumulation of liver or ectopic fat; or
- maintaining and/or improving the insulin sensitivity and/or for treating or preventing hyperinsulinemia and/or insulin resistance; or
- preventing, slowing progression of, delaying, or treating new onset diabetes after transplantation (NODAT) and/or post-transplant metabolic syndrome (PTMS); or
- preventing, delaying, or reducing NODAT and/or PTMS associated complications including micro- and macrovascular diseases and events, graft rejection, infection, and death; or
- treating hyperuricemia and hyperuricemia associated conditions.

In an alternative embodiment of the present disclosure, the disease comprises type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

According to another embodiment, the present disclosure also provides a method of treating type II diabetes mellitus by oral administration of a therapeutically effective amount of a crystal form of the present disclosure or a pharmaceutical composition thereof to a subject in need of the treatment. In one embodiment, the subject in need of the treatment is a human. In another embodiment, the pharmaceutical composition is in the form of a tablet.

According to another embodiment, the present disclosure also provides a method of administering to a subject one or more other combination drug treatments simultaneously or sequentially with the treatment with a therapeutically effective amount of a crystal form of the present disclosure, or a therapeutically effective amount of a pharmaceutical composition comprising the crystal form.

The compound of formula (A) or the pharmaceutical composition of the present disclosure can be administered once daily (QD), twice daily (BID) or three times daily (TID).

### EXAMPLES

The materials or reagents used herein are commercially available or prepared by synthetic methods commonly known in the art.

The following examples further describe and illustrate embodiments that are within the scope of the present disclosure. However, the present disclosure is not limited to the examples, and any modifications and replacements made on the technical basis of the present disclosure fall within the protective scope of the present disclosure.

In the following examples, the meanings of the abbreviations shown in Table 1 may be used.

**Table 1. Meanings of abbreviations**

| Abbreviation | Meaning |
|---|---|
| DMF | N,N-dimethylformamide |
| HATU | 2-(7-Azabenzotriazolyl)-N,N,N',N'-tetramethyluronium hexafluorophosphate |
| HMS5552 | (S)-2-(4-(2-chlorophenoxy)-2-oxo-2,5-dihydro-1H-pyrrol-1-yl)-N-(1-((R)-2,3-dihydroxypropyl)-1H-pyrazol-3-yl)-4-methylpentanamide |
| MeOH | Methanol |
| EtOH | Ethanol |
| IPA | Isopropyl alcohol |
| Acetone | Acetone |
| MIBK | Methyl isobutyl ketone |
| EtOAc | Ethyl acetate |
| IPAc | Isopropyl acetate |
| MTBE | Methyl tert-butyl ether |
| THF | Tetrahydrofuran |
| 2-MeTHF | 2-Methyltetrahydrofuran |
| Cyclohexane | Cyclohexane |
| 1,4-Dioxane | 1,4-Dioxane |
| ACN | Acetonitrile |
| DCM | Dichloromethane |
| CHCl₃ | Chloroform |
| Toluene | Toluene |
| n-Heptane | n-Heptane |
| DMSO | Dimethylsulfoxide |
| DMAc | N,N-dimethylacetamide |
| NMP | N-methyl-2-pyrrolidone |
| H₂O | Water |

### General identification methods for crystal forms

### 1. X-ray powder diffraction (XRPD)

An X-ray powder diffractometer produced by PANalytacal was used. The specific acquisition parameters were as shown in the table below.

| Model | Setting value |
|---|---|
| X-ray | Cu, Kα, Kα1 (Å): 1.540598, Kα2 (Å):1.544426 Kα2/Kα1 intensity ratio: 0.50 |
| Settings of X-ray tube | 45 kV, 40 mA |
| Divergence slit | 1/8° |
| Scan mode | Continuous |
| Scan range (°2Theta) | 3-40 |
| Scan time per step (s) | 46.7 |
| Step size (°2Theta) | 0.0263 |
| Testing time | About 5 min |

### 2. Thermogravimetric analysis (TGA) and differential scanning calorimetry (DCS)

A TA 5500 thermogravimetric analyzer was used to collect TGA, and a TA 2500 differential scanning calorimeter was used to collect DCS. The specific acquisition parameters were as shown in the table below.

| Parameter | TGA | DSC |
|---|---|---|
| Method | Linear temperature increase | Linear temperature increase |
| Sample pan | Aluminum pan, uncovered | Aluminum pan, covered/uncovered |
| Temperature range | Room temperature-an end-point temperature set by referring to a specific curve | 25°C-a set end-point temperature |
| Scan rate (°C/min) | 10 | 10 |
| Protective gas | Nitrogen | Nitrogen |

### 3. Modulated differential scanning calorimetry (mDSC)

A TA 2500 differential scanning calorimeter was used to collect mDSC. The specific acquisition parameters were as shown in the table below.

| Parameter | Setting value |
|---|---|
| Test mode | Conventional mDSC |
| Amplitude | 1.0°C |
| Modulation period | 60 s |
| Scan rate | 3.0 °C/min |
| Protective gas | Nitrogen, 50.00 mL/min |

### 4. Solution ¹H NMR

Solution ¹H NMR spectrum was collected on a Bruker 400M NMR instrument with DMSO-d6 as a solvent.

### 5. Ultra-performance liquid chromatography/ion chromatography (UPLC/IC)

In the test, the concentration was tested by Waters H-Class ultra-performance liquid chromatography system, and the salt-forming molar ratio of ions was tested by ion chromatography. The analysis conditions were as follows.

Test conditions of the ultra-performance liquid chromatography

| Liquid chromatography system | Waters H-Class, with PDA detector | |
|---|---|---|
| Column | Waters BEH C18, 50×2.1 mm, 1.7 µm | |
| | A: 0.1% TFA in H₂O | |
| Mobile phase | B: 0.1% TFA in ACN | |
| | Time (minutes) | %B |
| | 0.0 | 10 |
| | 3.0 | 90 |
| | 4.0 | 90 |
| | 4.1 | 10 |
| | 6.0 | 10 |
| Running time | 6.0 min | |
| Flow rate of the mobile phase | 1.0 mL/min | |
| Injection volume | 2 µL | |
| Detection wavelength | UV at 254 nm | |
| Column temperature | 40 °C | |
| Injector temperature | RT | |
| Diluent | ACN/H₂O (1:1, v/v) | |

Test conditions of the ion chromatography

| Ion chromatography system | ThermoFisher ICS-1100 |
|---|---|
| Column | IonPac AS18 Analytical Column, 250*4 mm |
| Mobile phase | 25 mM NaOH |
| Injection volume | 25 µL |
| Flow rate | 1.0 mL/min |
| Temperature | 35 °C |
| Column temperature | 35 °C |
| Current | 80 mA |
| Running time | 16 min |

### Example 1: Preparation of compound A

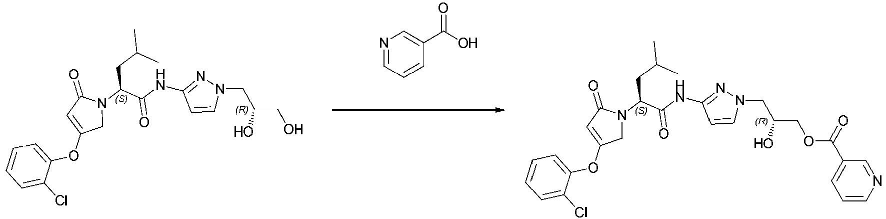

### Synthesis steps:

1.73 g (14.1 mmol, 1.3 eq.) of nicotinic acid was dissolved in DMF, and 4.19 g (32.4 mmol, 3.0 eq.) of diisopropylethylamine and 5.34 g of HATU (14.0 mmol, 1.3 eq.) were added at 0 °C. The reaction mixture was stirred at 0 °C for 30 min, and then 5.0 g (10.8 mmol, 1.0 eq.) of compound HMS5552 was added in batches. The reaction was stirred at room temperature for 20 h. The reaction mixture was quenched by adding water, and extracted three times with ethyl acetate. The organic phases were combined, washed with saturated brine, dried over anhydrous magnesium sulfate, and filtered. The filtrate was concentrated, and the residue was purified by preparative chromatography to give 3.3 g of **compound A** (53.7% yield, pink brown solid).

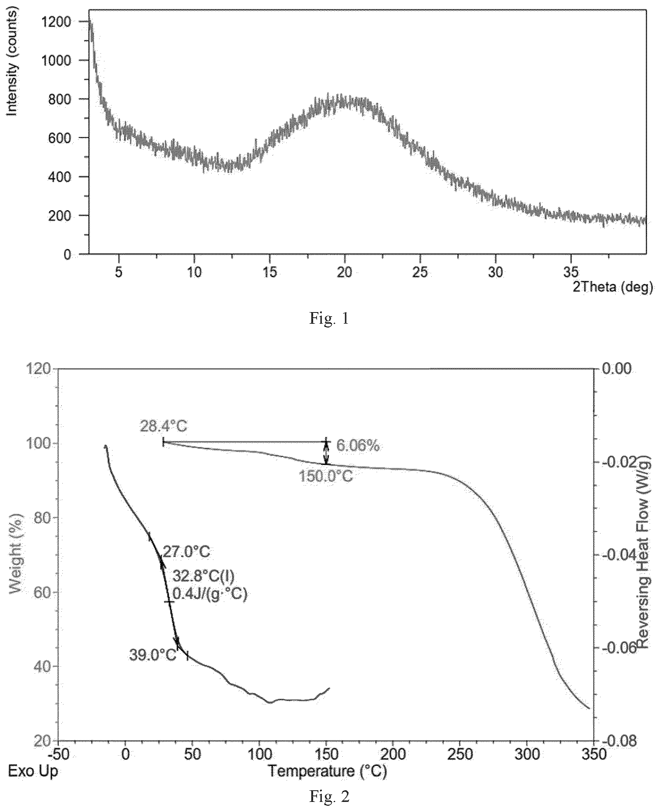

Compound A was subjected to X-ray powder diffraction and TGA/mDSC analysis (thermogravimetric analysis/modulated differential scanning calorimetry), and was found to be amorphous (Fig. 1). The TGA/mDSC results (Fig. 2) show that the sample has a weight loss of 6.06% when heated to 150 °C, and a glass transition temperature of 32.8 °C (intermediate temperature).

MS[M+H]⁺: 568.11; ¹H-NMR (d⁶-DMSO, 400 MHz): δ 10.81(s, 1H), 9.16(s, 1H), 8.82-8.84(d, J=4.52, 1H), 8.32-8.34(d, J=8.00, 1H), 7.62-7.66(t, J=8.78, 2H), 7.57-7.60(dd, J1=7.88, J2=4.68, 1H), 7.52-7.54(d, J=8.04, 1H), 7.45-7.49(t, J=7.72, 1H), 7.35-7.39(t, J=7.62, 1H), 6.45(s, 1H), 5.40-5.60(br, 1H), 4.89-4.92(dd, J1=10.60, J2=4.72, 1H), 4.80(s, 1H), 4.60-4.64(d, J=18.44, 1H), 4.012-4.29(m, 6H), 1.68-1.80(m, 1H), 1.48-1.62(m, 1H), 1.32-1.50(m, 1H), 0.94-0.95(d, J=6.52, 3H), 0.90-0.92(d, J=6.52, 3H).

### Example 2: Stability test of compound A in simulated gastric fluid (SGF)

**Preparation of simulated gastric fluid (SGF):** 0.04 g of NaCl and 0.064 g of pepsin were dissolved in 0.14 mL of HCl, and water was added to bring the total volume to 20 mL. The pH of the test solution was about 1.20±0.05.

**Preparation of compound A working solution:** 5 µL of 30 mM compound A stock solution was added to 745 µL of DMSO to yield 200 µM compound A working solution.
1) 2 µL of 200 µM compound A working solution was added into corresponding wells (n=2) of T0, T60, T120, T360, and T1440 of a 96-deep-well plate.
2) 198 µL of SGF solution was transferred to the above corresponding wells except T0 to reach a final test compound concentration of 2 µM for each time point (60, 120, 360, and 1440 minutes). The final concentration of DMSO in the incubation mixture was 1%.
3) Samples were incubated at 37 °C, 600 rpm for the appointed time.
4) Samples at corresponding incubation time point (60, 120, 360, and 1440 minutes) were removed and immediately mixed completely with 400 µL of cold acetonitrile containing 200 ng/mL tolbutamide (internal standard).
5) 200 µL of supernatant was removed and mixed completely again with 400 µL of cold acetonitrile containing 200 ng/mL tolbutamide (internal standard).
6) Preparation of the T0 samples: 198 µL of SGF solution was added to corresponding well, and mixed completely with 400 µL of cold acetonitrile containing 200 ng/mL tolbutamide (internal standard). Then 200 µL of supernatant was pipetted and mixed completely again with 400 µL of cold acetonitrile containing 200 ng/mL tolbutamide (internal standard).
7) All samples were centrifuged at 4000 rpm, 4 °C for 20 min.
8) 60 µL of supernatant was pipetted and mixed completely with 180 µL of ultra-pure water, and the mixture was subjected to LC-MS/MS analysis. The concentrations of the test compound and the converted compound HMS5552 were measured, and the retention rate/generation rate versus incubation time were plotted to evaluate the stability of the test compound in simulated gastric fluid (SGF).

LC-MS/MS condition:
LC: Shimadzu LC-30AD,
MS: QTRAP 6500+,
Autosampler: CTC PAL,
Mobile phase: A: 0.1% formic acid in water, B: 0.1% formic acid in acetonitrile,
Column: ACQUITY UPLC Protein BEH C4 300A 2.1*50 mm Part No.186004495,
Total Flow: 600 µL/min,
Scan Type: multiple reaction monitoring (MRM).

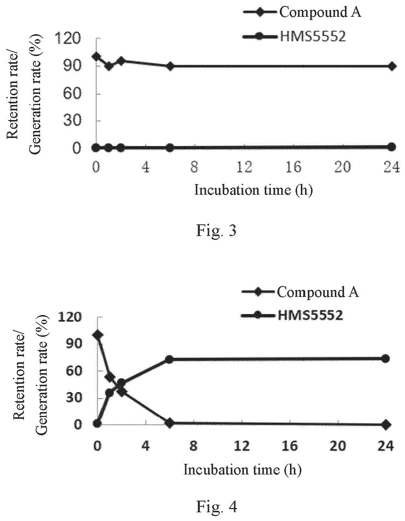

The results are shown in Table 2 and Fig. 3.

**Table 2 Stability data of compound A in simulated gastric fluid**

| | Time (hr) | Compound A (%) | Parent drug HMS5552 (%) |
|---|---|---|---|
| Compound A | 0 | 100.00 | 0.92 |
| | 1 | 90.35 | 0.86 |
| | 2 | 95.99 | 0.87 |
| | 6 | 90.10 | 1.02 |
| | 24 | 89.48 | 1.63 |

The results in Table 2 and Fig. 3 show that compound A was stable in SGF for 24 hours, and only a small amount of compound A was degraded into the parent drug HMS5552.

**Example 3: Stability test of compound A in simulated intestinal fluid (SIF)**

**Preparation of simulated intestinal fluid (SIF):** 0.136 g of KH₂PO₄ and 0.2 g of pancreatin were dissolved in water to obtain a final volume of 20 mL. The pH of the test solution was about 6.80±0.05.

**Preparation of compound A working solution:** 10 µL of 10 mM compound A stock solution was added to 490 µL of DMSO to obtain 200 µM compound A working solution.
1) 2 µL of 200 µM working solution was added into corresponding wells (n=2) of T0, T60, T120, T360, and T1440 of a 96-deep-well plate.
2) 198 µL of SIF solution was transferred to the above corresponding wells except T0 to reach a final test compound concentration of 2 µM for each time point (60, 120, 360, and 1440 minutes). The final concentration of DMSO in the incubation mixture was 1%.
3) Samples were incubated at 37 °C, 600 rpm for the appointed time.
4) Samples at corresponding incubation time point (60, 120, 360, and 1440 minutes) were removed and immediately mixed completely with 400 µL of cold acetonitrile containing 200 ng/mL tolbutamide (internal standard).
5) 200 µL of supernatant was removed and mixed completely again with 400 µL of cold acetonitrile containing 200 ng/mL tolbutamide (internal standard).
6) Preparation of the T0 samples: 198 µL of SIF solution was added to corresponding well, and mixed completely with 400 µL of cold acetonitrile containing 200 ng/mL tolbutamide (internal standard). Then 200 µL of supernatant was pipetted and mixed completely again with 400 µL of cold acetonitrile containing 200 ng/mL tolbutamide (internal standard).
7) All samples were centrifuged at 4000 rpm, 4 °C for 20 min.
8) 60 µL of supernatant was pipetted and mixed completely with 180 µL of ultra-pure water, and the mixture was subjected to LC-MS/MS analysis. The concentrations of compound A and the converted compound HMS5552 were measured, and the retention rate/generation rate versus incubation time were plotted to evaluate the stability of compound A in simulated intestinal fluid (SIF).

LC-MS/MS condition:
LC: Shimadzu LC 30-AD,
MS: API4000,
Autosampler: CTC PAL,
Mobile phase: A: 0.1% formic acid in water, B: 0.1% formic acid in acetonitrile,
Column: ACQUITY UPLC BEH C18 1.7µm 2.1 × 50mm Part No.186002350,
Scan Type: multiple reaction monitoring (MRM).

The results are shown in Table 3 and Fig. 4.

**Table 3 Stability data of compound A in simulated intestinal fluid**

| | Time (hr) | Compound A (%) | Parent drug HMS5552 (%) |
|---|---|---|---|
| | 0 | 100.00 | 0.86 |
| | 1 | 53.14 | 35.61 |
| Compound A | 2 | 37.56 | 46.64 |
| | 6 | 2.35 | 72.79 |
| | 24 | 0.00 | 73.43 |

The results in Table 3 and Fig. 4 show that compound A was extensively degraded in SIF, and most of compound A was converted to the parent drug HMS5552.

### Example 4: Simulated metabolic test of compound A in human intestine S9

**Preparation of phosphate buffer (PB):** 73.21 g of K₂HPO₄·3H₂O (AR grade) and 10.78 g of KH₂PO₄ (AR grade) were dissolved in ultra-pure water to obtain a final volume of 4000 mL and a final concentration of 100 mM. The pH of the final test solution was adjusted to 7.40±0.10 with H₃PO₄/KOH.

**Preparation of compound A working solution:** 5 µL of compound A stock solution (10 mM in DMSO) were diluted with 995 µL of acetonitrile (ACN) to give a compound A working solution (50 µM in 99% ACN)

The main materials of the test are shown in Table 4, and the parameters of preparation components of human intestinal S9 (HIS9) solution are shown in Table 5.

**Table 4 Main materials of the test**

| Materials | Product information | Source |
|---|---|---|
| Human intestine S9 (HIS9) | Cat. No.H0610.IS9 (NP) Lot No.1710039 | Xenotech |
| D-glucaric acid-1,4-lactone (100 mM) | S0375 | Sigma |

Table 5 The parameters of preparation components of human intestinal S9 solution

| Components of human intestinal S9 mixture | Volume (µL) | Concentration of compound A working solution | Final concentration |
|---|---|---|---|
| HIS9 (4 mg- protein/mL) | 675 | 1.0 mg/mL | 0.5mg/mL |
| D-glucaric acid-1,4-lactone (100 mM) | 270 | 10 mM | 5 mM |
| 100 mM PB | 1755 | - | - |
| Total volume | 2700 | - | - |

An Apricot automation workstation was used to add 50 µL/well of HIS9 solution to the corresponding wells of all reaction plates (blank, T0, T5, T15, T30, T45, T60).

Using an Apricot automation workstation, 2 µL/well of compound A working solution was added to the corresponding wells of all 96-well reaction plates except the blank (T0, T5, T15, T30, T45, T60).

An Apricot automation workstation was used to add 48 µL/well of PB to the corresponding wells of every reaction plate (blank, T0, T5, T15, T30, T45, T60) to start the reaction.

The reaction plates were incubated at 37 °C, and timer was started. An Apricot automation workstation was used to add 300 µL/well of stop solution (cold acetonitrile containing 200 ng/mL tolbutamide (internal standard)) to the corresponding wells of each reaction plate at the appropriate end time point to stop the reaction.

Each plate was sealed and shaken for 10 minutes. Each plate was centrifuged at 4000 rpm and 4 °C for 20 minutes. After centrifugation, 100 µL of supernatant was transferred from each reaction plate to the corresponding analysis plate, and mixed well with 300 µL of ultra-pure water.

Each analysis plate was sealed for LC-MS/MS analysis to obtain concentrations of compound A and the converted compound HMS5552 so as to assess the metabolic transformation of compound A in human intestine S9.
LC-MS/MS condition:
LC: Shimadzu LC 30-AD,
MS: API4000,
Autosampler: CTC PAL,
Mobile phase: A: 0.1% formic acid in water, B: 0.1% formic acid in acetonitrile,
Column: ACQUITY UPLC BEH C18 1.7µm 2.1x50mm Part No.186002350.
The results are shown in Table 6.

**Table 6 Simulation results of compound A in human intestine S9**

| | Time (min) | HIS9 | |
|---|---|---|---|
| | | Compound A (%) | Parent drug HMS5552 (%) |
| | 0 | 100 | 1.1 |
| | 5 | 0 | 80.0 |
| Compound A | 15 | 0 | 77.2 |
| | 30 | 0 | 79.8 |
| | 45 | 0 | 81.2 |
| | 60 | 0 | 90.5 |

Simulation results of human intestine S9 in Table 6 show that compound A was well metabolized in human intestine S9, and was completely metabolized and converted into the parent drug HMS5552 within 5 minutes.

### Example 5: Test using Caco-2 cells to bi-directionally assess the permeability of compound A

Caco-2 cell culture: Caco-2 cells (purchased from American Type Culture Collection, ATCC) were inoculated on polyethylene film (PET) of a 96-well culture plate at a concentration of 1x10⁵ cells/cm². The culture medium was replaced every 4 days until cell confluency to form cell monolayer on day 21 or 28.

Transport test: Transport buffer used in the test was Hanks' Balanced Salt Solution (HBSS) containing 10 mM 2-[4-(2-hydroxyethyl)-1-piperazinyl] ethanesulfonic acid (HEPES) with a pH of 7.40 ± 0.05. In vitro permeability studies were performed with Nadolol, Metoprolol and Digoxin as model permeants. Test compound A was assessed bi-directionally with test concentrations of 2.00, 10.0 and 30.0 µM (n=2), respectively. Digoxin was assessed bi-directionally with a test concentration of 10.0µM (n=2). Nadolol and Metoprolol were assessed unidirectionally both with a test concentration of 2.00 µM (n=2). The final concentration of DMSO in the incubation system was adjusted to <1%.

Specific steps:
1) The plate was placed in an incubator at 37±1°C with 5% CO₂ and saturated humidity, and incubated for 2 hours without shaking.
2) At the end of incubation, all samples were mixed with acetonitrile containing internal standard, and centrifuged at 3200 g for 10 minutes.
3) For compound A in groups 10.0 µM and 30.0µM, T0 sample and the donor side sample were diluted 10 times with blank sample supernatant.
4) For Nadolol and Metoprolol, 200 µL of supernatant was diluted with 600uL of ultra-pure water for LC-MS/MS analysis.
5) For Digoxin and test compound, 200 µL of supernatant was diluted with 200uL ultra-pure water for LC-MS/MS analysis.
6) The concentrations of compound A, the parent drug HMS5552 and the control compound in the initial solution, the donor side solution, and the receiver side solution were quantitatively determined by the peak area ratio of the tested substance to the internal standard by LC-MS/MS.

Permeability test results of the compound are shown in Table 7.

**Table 7 Permeability test results of compound A**

| Compound A concentratio n (µM) | The average apparent permeability Pₐₚₚ (10⁻⁶ cm/s) | | Efflux ratio | Average recovery rate (%) | | Average conversion rate to parent drug HMS5552 (%) | |
|---|---|---|---|---|---|---|---|
| | A→B | B→A | | A→B | B→A | A→B | B→A |
| 2.00 | 0.02977 | 11.9 | 401 | 52.9 | 87.1 | 26.5 | 5.8 |
| 10.0 | 0.03187 | 12.0 | 376 | 37.5 | 81.6 | 37.4 | 5.6 |
| 30.0 | 0.04322 | 11.9 | 276 | 37.6 | 82.2 | 26.8 | 3.4 |

Results in Table 7 show that the permeability of compound A from A side to B side is low, while the permeability of compound A from B side to A side is high, which suggested that compound A is likely to be the substrate for efflux transporters.

### Example 6: Pharmacokinetic Studies

There were 2 groups of SD male rats (HMS5552 group and compound A group, 6 rats in each group). Each group was divided into 2 subgroups (intravenous bolus group (IV) and gavage group (PO)). The intravenous bolus group (IV) was dosed 10 mg/kg HMS5552 or 10 mg/kg compound A by a single intravenous bolus injection. The gavage group (PO) was dosed 30 mg/kg HMS5552 or 30 mg/kg compound A by a single gavage.

Whole blood samples (about 0.2 mL per time point) were collected at pre-dose (0), 0.083, 0.25, 0.5, 1, 2, 4, 8, 12, and 24 hours post-dose by jugular vein puncture. All the blood samples were immediately transferred into labeled pre-chilled commercial microcentrifuge tubes containing 4 µL of 0.5M K₂-EDTA and 10 µL of cocktail stabilizer. Samples were placed on wet ice until centrifugation (within 30 min). Plasma samples were then prepared by centrifuging the blood samples at approximately 4 °C, 3200xg for 10 minutes. Supernatant plasma was pipetted, then quickly frozen over dry ice, and then kept at -60 °C or lower temperature until LC-MS/MS analysis. The cocktail stabilizer was prepared according to the following Table 8:

**Table 8 Cocktail stabilizer formula**

| Cocktail stabilizer components | Stock concentration of components | Stock solvent | Volume | Concentrati on of components | Ratio of cocktail stabilizer to whole blood sample | Final concentra tion of compone nts |
|---|---|---|---|---|---|---|
| Citric acid | 600 mM | Water | 0.5 mL | 150 mM | 1:20 (v:v), e.g., 0.1 mL of cocktail stabilizer+2.0 mL of whole blood | 7.5 mM |
| phenylmethyls ulfonyl fluoride (PMSF) | 400 mM | DMSO | 0.5 mL | 100 mM | | 5 mM |
| NaF | 400 mM | Water | 0.5 mL | 100 mM | | 5 mM |
| Dichlorvos | 400 mM | Water | 0.5 mL | 100 mM | | 5 mM |

The plasma concentrations were subjected to a non-compartmental pharmacokinetic analysis by using the WinNonlin^{™} software program (version 6.3). The linear/log trapezoidal rule was applied in obtaining the PK parameters, and the results are shown in Table 9.

**Table 9 Pharmacokinetic results**

| Group | Group #1 | | Group #2 | | | |
|---|---|---|---|---|---|---|
| Test compound | HMS5552 | | Compound A | | | |
| Route | IV (parent drug) | PO (parent drug) | IV (prodrug) | PO (prodrug) | IV (parent drug) | PO (parent drug) |
| Cmax (ng/mL) | / | 2459 | / | 0.973 | / | 1346 |
| Cₘₐₓ ratio (%, prodrug/parent drug) | / | / | / | 0.07 | / | / |
| Cₘₐₓ ratio (%, parent drug converted from prodrug/parent drug in group#1) | / | / | / | / | / | **67.2**** |
| Tₘₐₓ (h)* | / | 1 [1-1] | / | 0.25 [0.25-0.25] | / | 1 [0.5-1] |
| AUC₀₋ₗₐₛₜ (h·ng/mL) | 5049 | 7737 | 576 | 1.4 | 3422 | 5063 |
| AUC_{inf} (h·ng/mL) | 5051 | 7758 | 577 | 2.05 | 3423 | 5069 |
| AUC Ratio (%, prodrug/parent drug) | / | / | / | 0.04 | / | / |
| AUC Ratio (%, parent drug converted from prodrug/parent drug in group#1) | / | / | / | / | / | **80.2**** |
| CL (mL/min/kg) | 33.1 | / | 290 | / | / | / |
| V_{d} (L/kg) | 1.66 | / | 2.49 | / | / | / |
| T_{1/2} (h)* | 3.51 | 3.34 | 0.33 | / | 3.59 | 2.63 |
| Bioavailability (%) | / | 51.2 | / | 0.08 | / | / |

| | | | | | | |
|---|---|---|---|---|---|---|
| *: Tₘₐₓ is median [Min, Max]; **: converted based on molar (MW of HMS5552 and compound A are 462.93 and 568.03, respectively). | | | | | | |

The results in Table 9 show that compound A was metabolized very rapidly in rats by intravenous injection. When administered orally, the exposures (Cmax, Cmax ratio, AUC, AUC ratio) of compound A were comparable to those of the compound HMS5552. The above research results show that in rats, compound A is converted in the gastrointestinal tract and enterocytes into compound HMS5552, which is absorbed and then enters the circulatory system.

### Example 7: Pharmacodynamic studies

The glucose-lowering effect of compound HMS5552 and compound A were evaluated in C57BL/6J mice.

### Method

There were 7 groups of mice, with 8 mice in each group. Mice in group #1 were orally administered with vehicle as a control, and mice in other groups were orally administered with corresponding concentrations of compound A, with a dosage volume of 5 mL/kg. The low, medium, and high dose levels of compound A administration group were equivalent to (equimolar) the doses of HMS5552 at 5, 15, and 40 mg/kg. Oral glucose tolerance test (OGTT) was performed 1 hour after administration. The blood glucose level in mice was tested before administration (-60 min), before sugar administration (0 min), and at 15 min, 30 min, 60 min, and 120 min after sugar administration. In addition, 40 µl of blood was collected from the tail tip before sugar administration (0 min) and at 15 min after sugar administration to collect the plasma for an insulin level test.

### Data Processing and Statistical Analysis

All data was transferred into an Excel document. The blood glucose results were expressed in mg/dL and expressed as MEAN ± SEM. Differences between multiple groups were analyzed using Dunnett's method in one-way analysis of variance (ANOVA) or two-way analysis of variance (ANOVA) with Graphpad Prism 8 software for comparison, and the differences were considered statistically significant when the P value was less than 0.05.

### Results

Body weight: During the experiment, the body weight of the mice in each group was similar, and there was no significant difference between the groups.

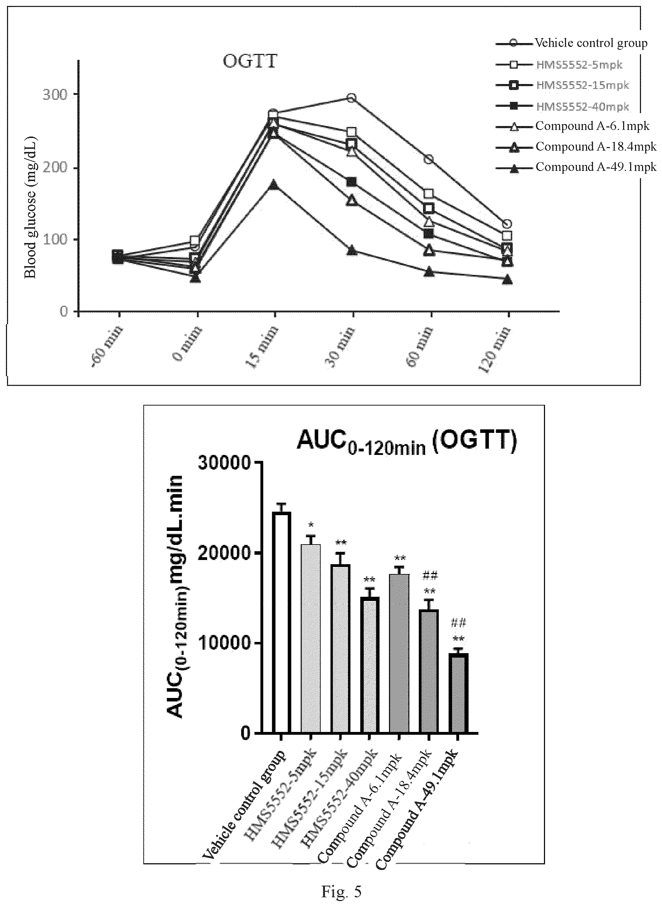

The OGTT results are shown in detail in Fig. 5. The blood glucose levels of the mice in each group at -60 min (pre-dose) were similar, and there was no significant difference between the groups. After administering compound HMS5552 and compound A to the mice, the blood glucose level of mice in each administration group at 0 min (pre-glucose) was generally lower than that of the vehicle control group, and there was a dose-response relationship. After glucose administration, the blood glucose values of mice in each group were increased significantly, wherein the blood glucose values of mice in the HMS5552-40mg/kg group were significantly lower than that of the vehicle control group at 30 min, 60 min, and 120 min. The blood glucose values of mice in compound A-49.1 mg/kg group were significantly lower than that of the vehicle control group at 15 min, 30 min, 60 min, and 120 min, and were significantly lower than that of the HMS5552-40mg/kg group at 30 min, 60 min, and 120 min. The AUC₀₋₁₂₀ₘᵢₙ of mice in each administration group was significantly lower than that of the vehicle control group, and there was a dose-response relationship. In addition, the AUC₀₋₁₂₀ₘᵢₙ of mice in the medium and high dose groups of compound A were significantly lower than those of the corresponding dose groups of HMS5552. The data shows that compound A has good glucose-lowering effect in mice.

The insulin levels of mice are shown in detail in Fig. 6. The insulin levels of mice in the vehicle control group at 0 min and 15 min were 0.13±0.02 (µg/L) and 0.50±0.05 (µg/L), respectively; the insulin levels of mice in each dose group of HMS5552 at 0 min and 15 min were slightly higher than those in the vehicle control group, without significant difference; at 0 min, the insulin level of mice in compound A high-dose group was significantly higher than that of the vehicle control group and the HMS5552 high-dose group; at 15 min, the insulin levels of mice in compound A-18.4mg/kg group and compound A-49.1mg/kg group were significantly higher than that of the vehicle control group, and the insulin level of compound A-18.4mg/kg group was significantly higher than that of the corresponding dose group of HMS5552.

This study indicates that compound A of the present disclosure has a good glucose-lowering effect in C57BL/6J mice. Compound A leads to increased insulin secretion and greater glucose-lowering effect as compared to HMS5552.

### Example 8: Solubility test of compound A starting material

A preliminary solubility test of the amorphous compound A starting material was carried out using 21 single solvents and 5 mixed solvents at room temperature (RT, 25±3 °C), including the following steps: about 2 mg of compound A starting material was weighed into a 3-mL vial, and then the corresponding solvent was gradually added until the solid was dissolved clearly; if the sample was still not dissolved clearly after adding 2 mL of the solvent, no more solvent was added. The solubility range of compound A in the corresponding solvent was calculated based on the mass of the sample and the volume of the added solvent, as shown in Table 10 below.

**Table 10 Solubility range of the starting material sample at room temperature**

| **Solvent** | **Solubility (mg/mL)** | **Solvent (v/v)** | **Solubility (mg/mL)** |
|---|---|---|---|
| MeOH | S>50.0 | DCM | S>44.0 |
| EtOH | S>46.0 | n-Heptane | S<1.1 |
| IPA | S>38.0 | Toluene | 20.0<S<40.0 |
| Acetone | S>46.0 | DMAc | S>42.0 |
| MIBK | S>40.0 | DMSO | S>44.0 |
| EtOAc | S>48.0 | NMP | S>44.0 |
| IPAc | S>44.0 | H₂O | S<1.1 |
| MTBE | 5.3<S<10.5 | Cyclohexane | S<1.1 |
| THF | S>40.0 | EtOH/H₂O (1:2) | 2.1<S<5.3 |
| 2-MeTHF | S>42.0 | Acetone/H₂O (1:2) | 5.3<S<10.5 |
| 1,4-Dioxane | S>42.0 | EtOAc/n-Heptane (1:4) | S<1.1 |
| ACN | S>50.0 | 2-MeTHF/n-Heptane (1:4) | S<1.0 |
| CHCl₃ | S>48.0 | Toluene/n-Heptane (1:1) | 1.0<S<2.0 |

### Example 9: Screening of free state crystal form of compound A

Based on the preliminary solubility results, the amorphous compound A was used as a starting material, and a total of 50 polymorph screening tests were set up using screening methods including suspension and stirring at room temperature, temperature cycling, slow cooling, slow evaporation, gas-liquid permeation, and antisolvent addition. The specific screening methods and results are summarized in Table 11. According to the XRPD results of the obtained solid, one free state crystal form (crystal form A) was found, which was determined to be an anhydrous crystal form based on the physical characterization results.

**Table 11 Summary of the polymorph screening test results**

| **Test method** | **Number of tests** | **Result** |
|---|---|---|
| Suspension and stirring at room temperature | 12 | Amorphous/gel |
| Temperature cycling | 8 | Amorphous/gel/oil |
| Slow cooling | 6 | Free state crystal form A/gel |
| Slow evaporation | 6 | Gel/oil |
| Gas-liquid permeation | 7 | Gel/oil |
| Antisolvent addition | 11 | Amorphous/gel |
| In total | 50 | Free state crystal form A/amorphous/gel/oil |

### Example 9.1: Suspension and stirring at room temperature

About 15 mg of the amorphous starting material in each portion was weighed into an HPLC vial, and 0.5 mL of a solvent listed in Table 12 was added, respectively. The resulting turbid liquid was magnetically stirred (1000 rpm) at room temperature for about 3 days, and then centrifuged to collect a solid. The solid was subjected to XRPD analysis. The test results are shown in Table 12. Amorphous and gel-forming samples were obtained.

**Table 12 Summary of the test of the suspension and stirring at room temperature**

| **Test No.** | **Solvent (v/v)** | **Result** |
|---|---|---|
| A1 | EtOH/H₂O (1:9) | Gel* |
| A2 | Acetone/n-Heptane (1:9) | Gel* |
| A3 | EtOAc/Cyclohexane (1:9) | Gel* |
| A4 | MTBE | Gel* |
| A5 | n-Heptane | Amorphous |
| A6 | Toluene/Cyclohexane (1:4) | Gel* |
| A7 | H₂O | Gel* |
| A8 | ACN/H₂O (1:9) | Gel* |
| A9 | DCM/n-Heptane (1:9) | Gel* |
| A10 | 2-MeTHF/n-Heptane (1:9) | Gel* |
| A11 | DMSO/H₂O (1:19) | Gel* |
| A12 | THF/n-Heptane (1:9) | Gel* |

| | | |
|---|---|---|
| *: The sample formed a gel when suspended and stirred at room temperature, and still formed a gel after being treated with temperature cycling. | | |

### Example 9.2: Temperature cycling

A total of 8 temperature cycling tests were set up using different solvent systems. About 15 mg of the amorphous starting material in each portion was weighed into a HPLC vial, and 0.5 mL of a solvent listed in Table 13 was added, respectively. The resulting suspension was magnetically stirred (1000 rpm) under temperature cycling (the sample was heated to 50 °C and then cooled to 5 °C at a rate of 0.1 °C/minute. The cycle was then repeated, and finally the sample was hold at 5 °C), and centrifuged to collect a solid. The solid was subjected to XRPD analysis. The test results are shown in Table 13. Amorphous, gel-forming and oil-forming samples were obtained.

**Table 13 Summary of the temperature cycling test**

| **Test No.** | **Solvent (v/v)** | **Result** |
|---|---|---|
| A1 | EtOH/n-Heptane (1:9) | Gel* |
| A2 | IPAc/n-Heptane (1:9) | Amorphous* |
| A3 | Acetone/H₂O (1:9) | Gel |
| A4 | MTBE/Cyclohexane (1:1) | Gel |
| A5 | THF/H₂O (1:9) | Gel |
| A6 | CHCl₃/n-Hexane (1:9) | Gel |
| A7 | DMAc/H₂O (1:9) | Gel |
| A8 | IPAc/H₂O (1:4) | Oil |

| | | |
|---|---|---|
| *: The sample was clear at 5 °C, and the final sample was obtained with stirring at -20 °C. | | |

### Example 9.3: Slow cooling

A total of 6 slow cooling tests were set up using different solvent systems. About 15 mg of the amorphous starting material in each portion was weighed into a 3-mL vial, and 1.5-3.0 mL of a solvent listed in Table 14 was added, respectively. The mixture was stirred and equilibrated at 50 °C for about 2 hours, and then filtered to obtain the supernatant. The resulting supernatant was placed in a biochemical incubator, which was cooled from 50 °C to 5 °C at a rate of 0.1 °C/min and then hold at a constant temperature of 5 °C. The precipitated solid was collected and subjected to XRPD analysis. The test results are shown in Table 14. Crystal form A of the free state and gel-forming samples were obtained in the slow cooling test.

**Table 14 Summary of the slow cooling test**

| **Test No.** | **Solvent (v/v)** | **Result** |
|---|---|---|
| A1 | EtOH/H₂O (1:2) | Gel* |
| A2 | Acetone/H₂O (1:2) | Gel^{#} |
| A3 | EtOAc/n-Heptane (1: 1) | Gel* |
| A4 | MTBE | Gel* |
| A5 | 2-MeTHF/n-Heptane (1:1) | Free state crystal form A** |
| A6 | Toluene/n-Heptane (1:1) | Gel* |

| | | |
|---|---|---|
| *: The sample had a small amount of oil droplets at 5 °C. After being placed at -20 °C, the liquid became turbid and gelatinous. Then, the sample was transferred to room temperature and exposed for slow evaporation. #: The sample was clear at 5 °C. Then, the sample was transferred to room temperature and exposed for slow evaporation. **: The sample was clear at 5 °C, and was clear after being placed at -20 °C. The sample was then transferred to room temperature and for slow evaporation with a pierced sealing film. | | |

### Example 9.4: Slow evaporation

A total of 6 slow evaporation tests were set up using different solvent systems. About 15 mg of the amorphous starting material in each portion was weighed into a 3-mL vial, and 0.2-3.0 mL of a solvent listed in Table 15 was added to dissolve the sample. The vial was sealed with a sealing film, and 4 pinholes were punched in the sealing film. The sample was placed at room temperature for slow evaporation. The resulting solid was collected and subjected to XRPD analysis. The test results are shown in Table 15. Gel-forming and oil-forming samples were obtained.

**Table 15 Summary of the slow evaporation test**

| **Test No.** | **Solvent (v/v)** | **Result** |
|---|---|---|
| A1 | MeOH/H₂O (4:1) | Gel |
| A2 | Acetone/n-Heptane (1:1) | Gel |
| A3 | MTBE | Gel |
| A4 | Toluene | Oil |
| A5 | 1,4-Dioxane/n-Heptane (1:1) | Oil |
| A6 | IPAc/Cyclohexane (1:1) | Gel |

### Example 9.5: Gas-liquid permeation

A total of 7 gas-liquid permeation tests were set up using different solvents. About 15 mg of the amorphous starting material in each portion was weighed into a 3-mL vial, and 0.4-0.5 mL of a solvent was added to dissolve the sample. Another 20-mL vial was taken, and about 3 mL of an antisolvent was added to it. The unsealed 3-mL vial with the clear liquid was placed in the 20-mL vial, and then the 20-mL vial was sealed and placed at room temperature. The resulting solid was collected and subjected to XRPD analysis. The test results are shown in Table 16. Gel-forming and oil-forming samples were obtained.

**Table 16 Summary of the gas-liquid permeation test**

| **Test No.** | **Solvent** | **Antisolvent** | **Result** |
|---|---|---|---|
| A1 | MIBK | n-Heptane | Gel* |
| A2 | Toluene | n-Heptane | Gel* |
| A3 | 1,4-Dioxane | Cyclohexane | Gel* |
| A4 | Toluene | Cyclohexane | Gel* |
| A5 | 1,4-Dioxane | H₂O | Gel* |
| A6 | NMP | H₂O | Oil^{#} |
| A7 | DMSO | H₂O | Gel* |

| | | | |
|---|---|---|---|
| *: The sample still formed a gel after being subjected to the temperature cycling treatment. #: The sample was clear and the product was obtained by slowly evaporating without sealing at room temperature. | | | |

### Example 9.6: Antisolvent addition

A total of 11 antisolvent addition tests were set up using different solvents. About 15 mg of the amorphous starting material in each portion was weighed into a 20-mL vial, and the solid was completely dissolved with 0.2 mL of a solvent (in Table 17). An antisolvent in Table 17 was added dropwise to the clear solution while stirring (1000 rpm) until a solid precipitated. Or, if there was no solid precipitated when the total volume of the added antisolvent reached 10 mL, the sample was suspended and stirred at 5 °C. The clear samples were transferred to - 20 °C, and suspended and stirred. The still clear samples were transferred to room temperature to evaporate. The precipitated solid was separated and subjected to XRPD analysis. The results are shown in Table 17. Amorphous and gel-forming samples were obtained in the antisolvent addition test.

**Table 17 Summary of the antisolvent addition test**

| **Test No.** | **Solvent** | **Antisolvent** | **Result** |
|---|---|---|---|
| A1 | IPA | n-Heptane | Gel* |
| A2 | IPA | H₂O | Amorphous |
| A3 | Acetone | n-Heptane | Gel* |
| A4 | Acetone | H₂O | Amorphous |
| A5 | IPAc | n-Heptane | Gel* |
| A6 | IPAc | Cyclohexane | Gel* |
| A7 | 2-MeTHF | n-Heptane | Gel* |
| A8 | 1,4-Dioxane | n-Heptane | Gel* |
| A9 | ACN | H₂O | Amorphous |
| A10 | NMP | H₂O | Amorphous |
| A11 | DMAc | H₂O | Amorphous |

| | | | |
|---|---|---|---|
| *: The sample still formed a gel after being subjected to the temperature cycling treatment. | | | |

In summary, examples 9.1 to 9.6 show that, in the 50 polymorph screening tests, crystal form A of the free state can only be obtained in the 2-MeTHF/n-Heptane (1:1, v/v) system after being slowly cooled, and then slowly evaporated at room temperature.

### Example 9.7: Characterization of crystal form A of the free state

### XRPD analysis

Fig. 7 shows the XRPD pattern of crystal form A of the free state of compound A, in which the diffraction angle 2θ°, d-spacing and relative intensity of the main peaks are listed in Table 18.

**Table 18 XRPD peak search data of crystal form A of the free state**

| **Diffraction angle [°2θ]** | **D-spacing [Å]** | **Relative intensity [%]** |
|---|---|---|
| 8.5364 | 10.36 | 97.61 |

| **Diffraction angle [°20]** | **D-spacing [Å]** | **Relative intensity [%]** |
|---|---|---|
| 10.0516 | 8.80 | 36.10 |
| 11.2306 | 7.88 | 13.83 |
| 12.0951 | 7.32 | 20.51 |
| 12.9134 | 6.86 | 25.90 |
| 14.1911 | 6.24 | 28.05 |
| 14.4798 | 6.12 | 36.26 |
| 15.2469 | 5.81 | 21.69 |
| 15.6676 | 5.66 | 7.36 |
| 17.7236 | 5.00 | 97.89 |
| 18.0204 | 4.92 | 100.00 |
| 18.5540 | 4.78 | 78.14 |
| 19.5544 | 4.54 | 12.92 |
| 20.1656 | 4.40 | 37.27 |
| 20.5977 | 4.31 | 45.32 |
| 21.2390 | 4.18 | 5.76 |
| 22.0149 | 4.04 | 25.10 |
| 23.1577 | 3.84 | 19.04 |
| 23.9360 | 3.72 | 21.60 |
| 25.0882 | 3.55 | 30.76 |
| 25.7424 | 3.46 | 33.74 |
| 28.6172 | 3.12 | 9.16 |
| 29.9813 | 2.98 | 6.56 |

### DSC and TGA

The TGA/DSC results (Fig. 8) show that the sample has a weight loss of 1.36% when heated to 150 °C, and has an endothermic peak at 135.5 °C (peak temperature). Since crystal form A of the free state has a small weight loss in TGA, and only a single endothermic signal is observed in the DSC curve, it is identified to be an anhydrous crystal form.

### Example 10: Screening of salt forms/cocrystals of compound A

The amorphous compound A was used as a starting material, and a total of 100 salt form/cocrystal screening tests were set up using 25 acids in 4 solvent systems. A total of 7 salt crystal forms and 1 free state crystal form (i.e., free state crystal form A) were obtained in the screening tests. All the salt forms were characterized by X-ray powder diffraction (XRPD), thermogravimetric analysis (TGA) and differential scanning calorimetry (DSC). And the salt-forming molar ratio was determined by solution-state ¹H NMR spectroscopy (¹H NMR) or ultra-performance liquid chromatography coupled with ion chromatography (UPLC/IC).

About 15 mg of the amorphous starting material and the corresponding acid in an equal molar ratio were mixed and stirred at room temperature for 3 days in 0.5 mL of a solvent. If a solid was obtained, the sample was centrifuged to separate the solid, and the solid was characterized by XRPD. The results are summarized in Table 19.

**Table 19 Summary of results of the salt form screening test**

| **No.** | **Acid** | **A: MTBE** | **B: Toluene** | **C: EtOAc/n-Heptane (1:4, v/v)** | **D: EtOH/n-Heptane (1:4, v/v)** |
|---|---|---|---|---|---|
| 1 | Hydrochloric acid | Hydrochloride crystal form A | Hydrochloride crystal form A | Hydrochloride crystal form A^{#} | Hydrochloride crystal form A* |
| 2 | Sulfuric acid | Gel** | Gel** | Gel** | Oil** |
| 3 | Aspartic acid | Gel** | Gel** | Gel** | Free state crystal form A** |
| 4 | Maleic acid | Gel** | Maleate crystal form B | Gel** | Oil** |
| 5 | Phosphoric acid | Gel** | Gel** | Gel** | Oil** |
| 6 | Glutamic acid | Free state crystal form A | Glutamic acid | Free state crystal form A ^{#} | Glutamic acid** |
| 7 | Mucic acid | Gel** | Mucic acid | Gel** | Gel** |
| 8 | Tartaric acid | Gel** | Gel** | Gel** | Oil** |
| 9 | Fumaric acid | Gel** | Gel** | Gel** | Fumaric acid^{#} |
| 10 | Citric acid | Gel** | Gel** | Gel** | Oil** |
| 11 | Malic acid | Gel** | Gel** | Gel** | Oil** |
| 12 | Hippuric acid | Gel** | Hippuric acid | Gel** | Free state crystal form A^{#} |
| 13 | Lactic acid | Gel** | Gel** | Gel** | Oil** |
| 14 | Ascorbic acid | Gel** | Gel** | Gel** | Gel** |
| 15 | Succinic acid | Free state crystal form A | Gel** | Gel** | Gel** |
| 16 | Adipic acid | Gel** | Gel** | Free state crystal form A** | Oil** |
| 17 | Sebacic acid | Gel** | Gel** | Gel** | Gel** |
| 18 | Acetic acid | Gel** | Gel** | Gel** | Oil** |
| 19 | 1.5-Naphthalenedi sulfonic acid | Amorphous * * | Naphthalene disulfonate crystal form A** | Gel** | Naphthalene disulfonate crystal form A** |
| 20 | p-Toluenesulfon ic acid | Gel** | Gel** | Gel** | Oil** |
| 21 | Methanesulfo nic acid | Gel** | Gel** | Gel** | Gel** |
| 22 | Benzenesulfo nic acid | Gel** | Gel** | Gel** | Gel** |
| 23 | Oxalic acid | Low crystallinity* | Oxalate crystal form A* | Oxalate crystal form A^{#} | Oil** |
| 24 | Benzoic acid | Gel** | Oil** | Gel** | Oil** |
| 25 | Hydrobromic acid | Hydrobromide crystal form A | Hydrobromide crystal form B | Hydrobromide crystal form B | Hydrobromide crystal form B |
| 26 | Blank | Gel** | Gel** | Gel** | Oil** |

| | | | | | |
|---|---|---|---|---|---|
| *: The sample slightly formed a gel after being stirred for 3 days, and the product was obtained after 10 days of temperature cycling treatment with stirring. #: The sample slightly formed a gel after being stirred for 3 days, and the product was obtained after 15 days of temperature cycling treatment with stirring. **: The sample slightly formed a gel after being stirred for 3 days, and the product was obtained after 19 days of temperature cycling treatment with stirring. | | | | | |

As shown in Table 19, a total of 7 salt crystal forms and 1 free state crystal form were obtained in the salt form screening tests. The obtained salt crystal forms and free state crystal form A were characterized by XRPD, TGA, DSC, ¹H NMR, or UPLC/IC.

### Example 10.1: Hydrochloride crystal form A

Crystal form A of the hydrochloride was obtained by stirring about 15 mg of the starting material and hydrochloric acid in an equal molar ratio in MTBE at room temperature for 3 days, followed by separating by centrifuging and then vacuum drying at room temperature for 18 hours.

### XRPD analysis

Fig. 9 shows the XRPD pattern of crystal form A of the hydrochloride of compound A, in which the diffraction angle 2θ°, d-spacing and relative intensity of the main peaks are listed in Table 20.

**Table 20 XRPD peak search data of crystal form A of the hydrochloride**

| **Diffraction angle [°20]** | **D-spacing [Å]** | **Relative intensity [%]** |
|---|---|---|
| 4.0636 | 21.74 | 86.11 |
| 8.1110 | 10.90 | 30.28 |
| 10.6058 | 8.34 | 36.71 |
| 11.8939 | 7.44 | 39.18 |
| 12.1781 | 7.27 | 22.96 |
| 15.2666 | 5.80 | 66.84 |
| 15.6573 | 5.66 | 19.52 |
| 16.2844 | 5.44 | 12.14 |
| 16.6797 | 5.32 | 19.99 |
| 17.8918 | 4.96 | 29.86 |
| 18.4527 | 4.81 | 100.00 |
| 19.4462 | 4.56 | 12.55 |
| 20.4121 | 4.35 | 15.66 |
| 21.5121 | 4.13 | 23.71 |
| 22.1424 | 4.01 | 32.60 |
| 22.9392 | 3.88 | 32.02 |
| 23.7499 | 3.75 | 5.78 |
| 24.9443 | 3.57 | 25.98 |
| 26.8884 | 3.32 | 21.10 |
| 27.5304 | 3.24 | 11.86 |
| 28.2329 | 3.16 | 9.77 |
| 30.6404 | 2.92 | 6.47 |

### ¹H NMR analysis

### The ¹H NMR data of crystal form A of the hydrochloride are as follows:

¹H NMR (400 MHz, d⁶-DMSO) δ 10.78(s, 1H), 9.20(d, *J*=2.08 Hz, 1H), 8.87-8.89(dd, *J₁*=5.00 Hz, *J*₂=1.68 Hz, 1H), 8.44-8.47(dt, *J₁*=8.00 Hz, *J*₂=2.00 Hz, 1H), 7.68-7.71(dd, *J₁*=7.96 Hz, *J₂*=5.04 Hz, 1H), 7.64-7.67(dd, *J₁*=8.00 Hz, *J₂*=1.40 Hz, 1H), 7.62-7.63(d, *J*=2.28 Hz, 1H), 7.51-7.54(dd, *J₁*=8.12 Hz, *J₂*=1.56 Hz, 1H), 7.45-7.49(td, *J₁*=7.68 Hz, *J*₂=1.39 Hz, 1H), 7.35-7.39(td, *J*₁*=7.64* Hz, *J*₂=1.93 Hz, 1H), 6.44(d, *J*=2.28 Hz, 1H), 4.88-4.92(dd, *J*₁=10.76 Hz, *J*₂=4.96 Hz, 1H), 4.80(s, 1H), 4.59-4.63(d, *J*=18.44 Hz, 1H), 4.12-4.30(m, ov, 7H), 1.73-1.82(m, 1H), 1.56-1.60(m, 1H), 1.41-1.48(m, 1H), 0.93-0.95(d, *J*=6.56 Hz, 3H), 0.90-0.91(d, *J*=6.56 Hz, 3H).

### DSC and TGA

The TGA/DSC results (Fig. 10) show that the sample has a weight loss of 4.91% when heated to 150 °C, and has 2 endothermic peaks at 125.9 °C and 177.8 °C (peak temperature). The crystalline form of crystal form A of the hydrochloride does not change after being heated to 100 °C, and crystal form A of the hydrochloride has a small weight loss in TGA from room temperature to 80 °C, so crystal form A of the hydrochloride is identified to be an anhydrous crystal form.

### UPLC/IC

The UPLC/IC results show that the molar ratio of crystal form A of the hydrochloride is 1.0 (acid/free state). The IC results show that the chloride ion content in the sample after being heated to 150 °C is 1.88wt%, and the corresponding acid-base molar ratio is calculated to be 0.3. Therefore, it is inferred that the weight loss in TGA from 80 °C to 150 °C corresponds to a deacidification signal.

### Example 10.2: Maleate crystal form A

Crystal form A of the maleate was obtained by stirring about 15 mg of the starting material and maleic acid in an equal molar ratio in toluene at room temperature for 3 days.

*XRPD analysis*

Fig. 11 shows the XRPD pattern of crystal form A of the maleate of compound A, in which the diffraction angle 2θ°, d-spacing and relative intensity of the main peaks are listed in Table 21.

**Table 21 XRPD peak search data of crystal form A of the maleate**

| **Diffraction angle [°2θ]** | **D-spacing [Å]** | **Relative intensity [%]** |
|---|---|---|
| 6.0101 | 14.71 | 100.00 |
| 6.9189 | 12.78 | 12.62 |
| 10.4123 | 8.50 | 18.64 |
| 12.0135 | 7.37 | 12.17 |
| 15.9271 | 5.56 | 17.37 |
| 17.3842 | 5.10 | 30.76 |
| 18.4174 | 4.82 | 57.57 |
| 19.4609 | 4.56 | 8.06 |
| 21.2127 | 4.19 | 12.07 |
| 21.7575 | 4.08 | 14.37 |
| 22.9314 | 3.88 | 5.12 |

### Example 10.3: Maleate crystal form B

The above crystal form A of the maleate was vacuum dried at room temperature for 18 hours to transform into a new crystal form, which was named as maleate crystal form B.

### XRPD analysis

Fig. 12 shows the XRPD pattern of crystal form B of the maleate of compound A, in which the diffraction angle 2θ°, d-spacing and relative intensity of the main peaks are listed in Table 22.

**Table 22 XRPD peak search data of crystal form B of the maleate**

| **Diffraction angle [°2θ]** | **D-spacing [Å]** | **Relative intensity [%]** |
|---|---|---|
| 4.0753 | 21.68 | 79.26 |
| 8.1265 | 10.88 | 94.14 |
| 10.5018 | 8.42 | 21.46 |
| 12.1989 | 7.26 | 27.72 |
| 13.3130 | 6.65 | 13.41 |
| 15.0103 | 5.90 | 21.08 |
| 16.2444 | 5.46 | 30.99 |
| 16.6849 | 5.31 | 72.32 |
| 17.5445 | 5.06 | 60.06 |
| 18.3734 | 4.83 | 12.47 |
| 20.1676 | 4.40 | 100.00 |
| 20.4062 | 4.35 | 34.05 |
| 21.3429 | 4.16 | 67.40 |
| 22.4006 | 3.97 | 34.55 |
| 22.6907 | 3.92 | 26.72 |
| 23.1628 | 3.84 | 13.72 |
| 24.1386 | 3.69 | 49.59 |
| 24.5568 | 3.63 | 30.91 |
| 25.9706 | 3.43 | 21.67 |
| 26.5459 | 3.36 | 34.49 |
| 27.5939 | 3.23 | 20.49 |
| 29.8963 | 2.99 | 15.45 |

The ¹H NMR spectrum shows that the molar ratio of acid to free state in crystal form B of the maleate is 1.2, and the molar ratio of toluene to API is 0.2 (about 2.2 wt%)

### DSC and TGA

The TGA/DSC results (Fig. 13) show that the sample has a weight loss of 12.0% when heated to 120 °C, and has an endothermic peak at 85.6 °C (peak temperature).

### Example 10.4: Naphthalene disulfonate crystal form A

Crystal form A of the naphthalene disulfonate was obtained by stirring about 15 mg of the starting material and naphthalene disulfonate in an equal molar ratio in toluene at room temperature for 3 days, and then undergoing temperature cycling treatment, followed by separating by centrifuging and then vacuum drying at room temperature for 2 hours.

### XRPD analysis

Fig. 14 shows the XRPD pattern of crystal form A of the naphthalene disulfonate of compound A, in which the diffraction angle 2θ°, d-spacing and relative intensity of the main peaks are listed in Table 23.

**Table 23 XRPD peak search data of crystal form A of the naphthalene disulfonate**

| **Diffraction angle [°2θ]** | **D-spacing [Å]** | **Relative intensity [%]** |
|---|---|---|
| 4.0252 | 21.95 | 100.00 |
| 10.2761 | 8.61 | 31.90 |
| 11.9617 | 7.40 | 12.57 |
| 13.6808 | 6.47 | 67.56 |
| 15.4877 | 5.72 | 28.71 |
| 20.9613 | 4.24 | 22.40 |
| 22.2682 | 3.99 | 28.03 |
| 26.2169 | 3.40 | 15.07 |

The ¹H NMR spectrum shows that the molar ratio of acid to base in crystal form A of the naphthalene disulfonate is 1.0, and the molar ratio of residual toluene to API is 0.02 (0.3 wt%).

### DSC and TGA

The TGA/DSC results (Fig. 15) show that the sample has a weight loss of 6.0% when heated to 150 °C, and has 2 endothermic peaks at 119.2 °C and 207.9 °C (peak temperature).

### Example 10.5: Oxalate crystal form A

Crystal form A of the oxalate was obtained by stirring about 15 mg of the starting material and oxalic acid in an equal molar ratio in toluene at room temperature for 3 days, and then undergoing the treatment of temperature cycling with stirring for 10 days, followed by separating by centrifuging and then vacuum drying at room temperature for 3 hours.

### XRPD analysis

Fig. 16 shows the XRPD pattern of crystal form A of the oxalate of compound A, in which the diffraction angle 2θ°, d-spacing and relative intensity of the main peaks are listed in Table 24.

**Table 24 XRPD peak search data of crystal form A of the oxalate**

| **Diffraction angle [°20]** | **D-spacing [Å]** | **Relative intensity [%]** |
|---|---|---|
| 5.0018 | 17.67 | 100.00 |
| 7.3700 | 12.00 | 35.55 |
| 15.0480 | 5.89 | 60.46 |
| 16.5219 | 5.37 | 41.72 |
| 17.3923 | 5.10 | 12.72 |
| 18.6326 | 4.76 | 8.67 |
| 19.3099 | 4.60 | 27.42 |
| 20.0972 | 4.42 | 28.35 |
| 20.6792 | 4.30 | 13.32 |
| 22.7159 | 3.91 | 14.72 |
| 25.2083 | 3.53 | 25.85 |
| 26.0154 | 3.43 | 21.20 |

### DSC and TGA

The TGA/DSC results (Fig. 17) show that the sample has a weight loss of 3.9% when heated to 150 °C, and has 2 endothermic peaks at 125.4 °C (starting temperature) and 192.8 °C (peak temperature).

### UPLC/IC

The UPLC/IC results show that the molar ratio of crystal form A of the oxalate is 1.1 (ligand/free state).

### Example 10.6: Hydrobromide crystal form A

Crystal form A of the hydrobromide was obtained by stirring about 15 mg of the starting material and hydrobromic acid in an equal molar ratio in MTBE at room temperature for 3 days. After being vacuum dried at room temperature for 18 hours, crystal form A of the hydrobromide turned into crystal form A of the free state.

### XRPD analysis

Fig. 18 shows the XRPD pattern of crystal form A of the hydrobromide of compound A, in which the diffraction angle 2θ°, d-spacing and relative intensity of the main peaks are listed in Table 25.

**Table 25 XRPD peak search data of crystal form A of the hydrobromide**

| **Diffraction angle [°2θ]** | **D-spacing [Å]** | **Relative intensity [%]** |
|---|---|---|
| 4.2552 | 20.77 | 42.66 |
| 8.2858 | 10.67 | 100.00 |
| 10.6343 | 8.32 | 5.38 |
| 12.4183 | 7.13 | 29.34 |
| 14.8106 | 5.98 | 19.32 |
| 16.5297 | 5.36 | 11.41 |
| 16.8559 | 5.26 | 19.87 |
| 17.5323 | 5.06 | 26.90 |
| 17.9040 | 4.95 | 22.59 |
| 20.4993 | 4.33 | 32.34 |
| 21.3131 | 4.17 | 35.04 |
| 22.5235 | 3.95 | 9.58 |
| 22.9553 | 3.87 | 9.11 |
| 24.6041 | 3.62 | 9.81 |
| 24.9613 | 3.57 | 12.75 |

### Example 10.7: Hydrobromide crystal form B

Crystal form B of the hydrobromide was obtained by stirring about 15 mg of the starting material and hydrobromic acid in an equal molar ratio in toluene at room temperature for 3 days, followed by separating by centrifuging and then vacuum drying at room temperature for 18 hours.

### XRPD analysis

Fig. 19 shows the XRPD pattern of crystal form B of the hydrobromide of compound A, in which the diffraction angle 2θ°, d-spacing and relative intensity of the main peaks are listed in Table 26.

**Table 26 XRPD peak search data of crystal form B of the hydrobromide**

| **Diffraction angle [°2θ]** | **D-spacing [Å]** | **Relative intensity [%]** |
|---|---|---|
| 4.0548 | 21.79 | 100.00 |
| 8.0954 | 10.92 | 69.38 |
| 10.5733 | 8.37 | 17.29 |
| 12.1371 | 7.29 | 19.94 |
| 12.7400 | 6.95 | 16.67 |
| 13.5450 | 6.54 | 14.61 |
| 15.0489 | 5.89 | 74.19 |
| 15.4264 | 5.74 | 18.36 |
| 16.2325 | 5.46 | 19.77 |
| 17.8422 | 4.97 | 27.20 |
| 18.4470 | 4.81 | 82.75 |
| 19.2677 | 4.61 | 12.01 |
| 20.3453 | 4.37 | 37.70 |
| 21.3334 | 4.17 | 24.51 |
| 22.0226 | 4.04 | 33.60 |
| 22.7663 | 3.91 | 32.00 |
| 23.6534 | 3.76 | 12.83 |
| 24.4377 | 3.64 | 54.97 |
| 26.8442 | 3.32 | 14.52 |
| 30.6471 | 2.92 | 12.88 |

### DSC and TGA

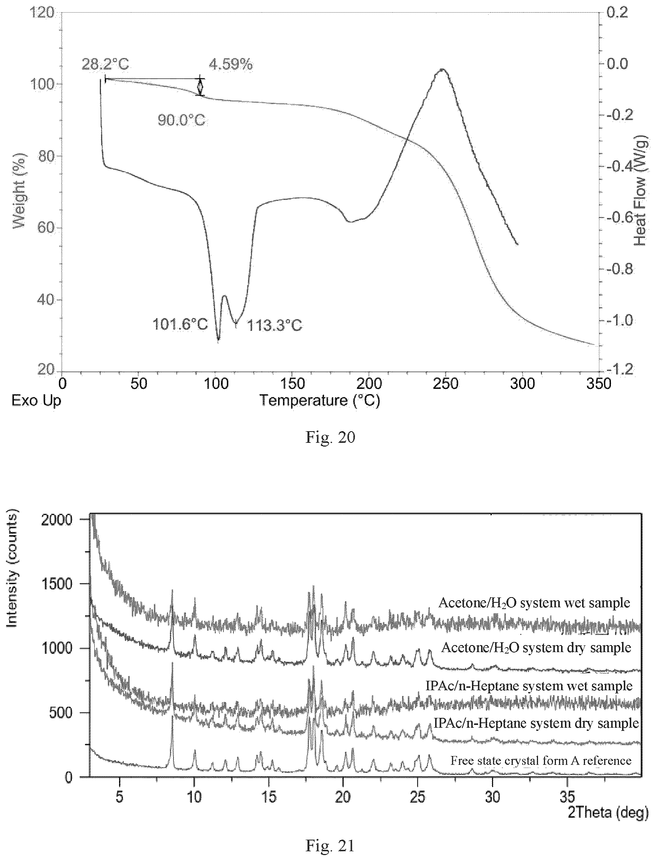

The TGA/DSC results (Fig. 20) show that the sample has a weight loss of 4.6% when heated to 90 °C, and has 2 endothermic peaks at 101.6 and 113.3 °C (peak temperature), respectively.

### UPLC/IC

The UPLC/IC results show that the molar ratio of crystal form B of the hydrobromide is 1.0 (acid/free state).

### Example 11: Repeated preparation of free state crystal form A

As described in Example 10, in the salt form (cocrystal) screening tests, crystal form A of the free state was also obtained by stirring compound A starting material and succinic acid in an equal molar ratio in MTBE at room temperature for 3 days, followed by separating by centrifuging and then vacuum drying at room temperature for 18 hours. Therefore, this method was used to repeatedly prepare crystal form A of the free state.

Specifically, about 105 mg of the starting material and succinic acid in an equal molar ratio were stirred in MTBE at room temperature for 3 days, separated by centrifuging, and then vacuum dried at room temperature for 3 hours to obtain the product.

The ¹H NMR spectrum shows that no succinic acid signal is detected in crystal form A of the free state, and the molar ratio of residual MTBE to API is 0.08 (1.2 wt%).

In addition, in order to study the process developability of crystal form A of the free state, an attempt was made to prepare crystal form A of the free state through solution crystallization.

Specifically, about 50 mg of the amorphous compound A starting material was weighed into a 20 mL vial, and the corresponding solvent in Table 27 was added to dissolve the solid with sonication. A total of about 5.0 mg of seed crystals of crystal form A of the free state was added into the vial, and an antisolvent was added dropwise until a solid precipitated. The sample was suspended and stirred at room temperature for another 3 hours, and the wet sample was subjected to XRPD analysis. The sample was stirred overnight, and then separated by centrifuging and dried at room temperature without sealing. The dry sample was subjected to XRPD analysis. The results are summarized in Table 27, and an overlaid XRPD pattern is shown in Fig. 21. The results show that crystal form A of the free state was obtained in both Acetone/H₂O and IPAc/n-Heptane systems.

**Table 27 Summary of results of the preparation of free state crystal form A by solution crystallization**

| **Test No.** | **Batch** | **Solvent (volume/mL)** | **Antisolvent (volume/mL)** | **Phenome Result non** | |
|---|---|---|---|---|---|
| A1 | 50 mg | Acetone (0.4) | H₂O (1.2) | Gel-forming* | Free state crystal form A |
| A2 | 50 mg | IPAc (0.6) | n-Heptane (2.0) | Gel-forming* | Free state crystal form A |

| | | | | | |
|---|---|---|---|---|---|
| *: After being stirred at room temperature for 3 hours, the sample turned into a suspension. | | | | | |

### Example 12: Screening of other free state crystal forms

Further polymorph screening of compound A was carried out using crystal form A of the free state as a starting material, to examine whether other potential crystal forms could be obtained, and to determine the interconversion relationship.

Specifically, a total of 52 polymorph screening tests were set up, using screening methods including, gas-solid permeation, suspension and stirring at room temperature and 50 °C, temperature cycling with stirring, and grinding. Based on the X-ray powder diffraction (XRPD) results of the isolated solid, no new crystal form was found.

### Example 12.1: Solubility test of free state crystal form A

A preliminary solubility test of the free state crystal form A starting material sample was carried out using 20 single solvents at room temperature (RT, 25±3 °C), including the following steps: about 2 mg of the starting material sample was weighed into a 3-mL vial, and then the corresponding solvent was gradually added until the solid was dissolved clearly; if the sample was still not dissolved clearly after adding 2 mL of the solvent, no more solvent was added. The solubility range in the corresponding solvent was calculated based on the mass of the sample and the volume of the added solvent, as shown in Table 28. The data were used as a base to guide the selection of solvent in screening tests.

**Table 28 Preliminary solubility of crystal form A of the free state at room temperature**

| **Solvent** | **Solubility (mg/mL)** | **Solvent** | **Solubility (mg/mL)** |
|---|---|---|---|
| MeOH | S>42.0 | 1,4-Dioxane | S>40.0 |
| EtOH | 20.0<S<40.0 | ACN | 10.0<S<20.0 |
| IPA | 5.0<S<10.0 | CHCl₃ | S>40.0 |
| Acetone | S>40.0 | DCM | S>40.0 |
| MIBK | 5.0<S<10.0 | n-Heptane | S<1.1 |
| EtOAc | 20.0<S<40.0 | Toluene | S<1.0 |
| IPA_{C} | 5.0<S<10.0 | DMAc | S>40.0 |
| MTBE | S<1.0 | DMSO | S>42.0 |
| THF | S>40.0 | NMP | S>40.0 |
| 2-MeTHF | 20.0<S<40.0 | H₂O | S<1.0 |

### Example 12.2: Polymorph screening

Crystal form A of the free state was used as a starting material, and a total of 52 polymorph screening tests were set up using screening methods including, gas-solid permeation, suspension and stirring at room temperature and 50 °C, temperature cycling with stirring, and grinding. The specific test methods and results are summarized in Table 29.

**Table 29 Summary of the polymorph screening test results**

| **Test method** | **Number of tests** | **Result** |
|---|---|---|
| Gas-solid permeation | 8 | Free state crystal form A/gel |
| Suspension and stirring at room temperature | 17 | Free state crystal form A/gel |
| Suspension and stirring at 50°C | 13 | Free state crystal form A |
| Temperature cycling with stirring | 12 | Free state crystal form A |
| Grinding | 2 | Free state crystal form A |
| In total | 52 | Free state crystal form A/gel |

### Example 12.2.1: Gas-solid permeation

A total of 8 gas-solid permeation tests were set up using different solvents. About 20 mg of crystal form A of the free state was weighed into a 3-mL vial, and about 3.0 mL of a solvent listed in Table 30 was added to a 20-mL vial. The unsealed 3-mL vial was placed in the 20-mL vial, and the 20-mL vial was sealed. After standing at room temperature for 6 days, a solid was collected and subjected to XRPD analysis. The test results are shown in Table 30. Gels and free state crystal form A were obtained.

**Table 30 Summary of the gas-solid permeation test**

| **Test No.** | **Solvent** | **Result** |
|---|---|---|
| A1 | EtOH | Free state crystal form A |
| A2 | Acetone | Free state crystal form A |
| A3 | EtOAc | Free state crystal form A |
| A4 | THF | Gel* |
| A5 | H₂O | Free state crystal form A |
| A6 | Toluene | Free state crystal form A |
| A7 | 1,4-Dioxane | Gel* |
| A8 | DMSO | Free state crystal form A |

| | | |
|---|---|---|
| *: The sample was dissolved clearly after being placed at room temperature for 3 days, and then slowly evaporated at room temperature to obtain a gel-forming sample. | | |

### Example 12.2.2: Suspension and stirring at room temperature

A total of 17 tests of suspension and stirring at room temperature were set up using different solvent systems. About 20 mg of crystal form A of the free state was weighed into an HPLC glass vial, and 0.5 mL of a solvent listed in Table 31 was added, respectively. The resulting turbid liquid was magnetically stirred (1000 rpm) at room temperature for about 3 days, and then centrifuged to collect a solid. The solid was subjected to XRPD analysis. The test results are shown in Table 31. Gels and free state crystal form A were obtained.

**Table 31 Summary of the test of suspension and stirring at room temperature**

| **Test No.** | **Solvent (v/v)** | **Result** |
|---|---|---|
| A1 | IPA | Free state crystal form A |
| A2 | IPAc | Free state crystal form A |
| A3 | MTBE | Free state crystal form A |
| A4 | MIBK | Free state crystal form A |
| A5 | n-Heptane | Free state crystal form A |
| A6 | Toluene | Free state crystal form A |
| A7 | H₂O | Free state crystal form A |
| A8 | MeOH/H₂O (1:9) | Free state crystal form A |
| A9 | Acetone/n-Heptane (1:9) | Free state crystal form A |
| A10 | ACN/H₂O (1:1) | Gel* |
| A11 | DCM/n-Heptane (1:9) | Free state crystal form A |
| A12 | 2-MeTHF/MTBE (1:9) | Free state crystal form A |
| A13 | DMSO/H₂O (1:9) | Free state crystal form A |
| A14 | IPA/H₂O (982:18, water activity a_{w}~0.2) | Gel^{#} |
| A15 | IPA/H₂O (957:43, water activity a_{w} ~0.4) | Gel^{#} |
| A16 | IPA/H₂O (918:82, water activity a_{w} ~0.6) | Gel ^{#} |
| A17 | IPA/H₂O (849:151, water activity a_{w} ~0.8) | Gel ^{#} |

| | | |
|---|---|---|
| *: The sample was stirred at room temperature for 2 days to form an oil, then stirred at 5 °C for 1 day to form an oil. The sample was then slowly evaporated at room temperature to obtain a gel-forming sample. #: The sample was clear after being stirred at room temperature for 2 days, and was clear after being stirred at 5 °C for 1 day. The sample was then slowly evaporated at room temperature to obtain a gel-forming sample. | | |

### Example 12.2.3: Suspension and stirring at 50 °C

A total of 13 tests of suspension and stirring at 50 °C were set up using different solvent systems. About 20 mg of crystal form A of the free state was weighed into an HPLC glass vial, and 0.5 mL of a solvent listed in Table 32 was added, respectively. The resulting suspension was magnetically stirred (1000 rpm) at 50 °C for about 3 days, and then centrifuged to collect a solid. The solid was subjected to XRPD analysis. The test results are shown in Table 32. Crystal form A of the free state was obtained.

**Table 32 Summary of the test of suspension and stirring at 50°C**

| **Test No.** | **Solvent (v/v)** | **Result** |
|---|---|---|
| A1 | EtOH/H₂O (1:4) | Free state crystal form A |
| A2 | IPA | Free state crystal form A* |
| A3 | MIBK | Free state crystal form A |
| A4 | MTBE | Free state crystal form A |
| A5 | n-Heptane | Free state crystal form A |
| A6 | H₂O | Free state crystal form A |
| A7 | 2-MeTHF/n-Heptane (1:4) | Free state crystal form A |
| A8 | EtOAc/Toluene (1:4) | Free state crystal form A |
| A9 | ACN/MTBE (1:1) | Free state crystal form A* |
| A10 | 1,4-Dioxane/MTBE (1:9) | Free state crystal form A |
| A11 | THF/Toluene (1:9) | Free state crystal form A |
| A12 | CHCl₃/n-Heptane (1:9) | Free state crystal form A |
| A13 | DMAc/H₂O (1:9) | Free state crystal form A |

| | | |
|---|---|---|
| *: The sample was clear after being stirred at 50 °C for 3 days, and then transferred to 5 °C and stirred for 1 day to precipitate solid. | | |

### Example 12.2.4: Temperature cycling with stirring

A total of 12 tests of temperature cycling with stirring were set up using different solvent systems. About 20 mg of crystal form A of the free state was weighed into an HPLC glass vial, and 0.5 mL of a solvent listed in Table 33 was added, respectively. The resulting suspension was magnetically stirred (1000 rpm) under temperature cycling (the sample was heated to 50°C and then cooled to 5 °C at a rate of 0.1 °C/minute. The cycle was then repeated, and finally the sample was maintained at 5°C), and centrifuged to collect a solid. The solid was subjected to XRPD analysis. The test results are shown in Table 33. Crystal form A of the free state was obtained.

**Table 33 Summary of the test of temperature cycling with stirring**

| **Test No.** | **Solvent (v/v)** | **Result** |
|---|---|---|
| A1 | MeOH/MTBE (1:4) | Free state crystal form A |
| A2 | IPA | Free state crystal form A |
| A3 | MTBE | Free state crystal form A |
| A4 | IPAc | Free state crystal form A |
| A5 | ACN | Free state crystal form A |
| A6 | Toluene | Free state crystal form A |
| A7 | MIBK | Free state crystal form A |
| A8 | THF/H₂O (1:9) | Free state crystal form A |
| A9 | NMP/H₂O (1:9) | Free state crystal form A |
| A10 | EtOAc/n-Heptane (1:4) | Free state crystal form A |
| A11 | THF/MTBE (1:9) | Free state crystal form A |
| A12 | Acetone/n-Heptane (1:4) | Free state crystal form A |

### Example 12.2.5: Grinding

A total of 2 grinding tests were set up under different conditions. About 20 mg of crystal form A of the free state was weighed into a mortar, and 20 µL of a solvent listed in Table 34 was added, respectively. The sample was grinded manually for 3 to 5 minutes. The resulting solid was collected and subjected to XRPD analysis. The test results are shown in Table 34. Crystal form A of the free state was obtained.

**Table 34 Summary of the grinding test**

| **Test No.** | **Solvent** | **Result** |
|---|---|---|
| A1 | N/A | Free state crystal form A |
| A2 | H₂O | Free state crystal form A |

In summary, a total of 52 polymorph screening tests were set up using crystal form A of the free state as a starting material. According to the XRPD results of the isolated solids, no new crystal form was found.

### Example 13: Preparation of hydrochloride crystal form B

5 g of compound A was dissolved in 100 mL of tetrahydrofuran, and 2 mL of 6N hydrochloric acid was slowly added dropwise at room temperature. The mixture was stirred at room temperature overnight, filtered, washed with tetrahydrofuran, and dried to give crystal form B of a hydrochloride of compound A with a yield of 98%.

### Example 14: Further screening of hydrochloride crystal forms

Crystal form B of the hydrochloride of compound A was used as a starting material, and a total of 50 polymorph screening tests were set up using screening methods including, slow evaporation, temperature cycling with stirring, grinding, suspension and stirring at room temperature and 50 °C, and antisolvent addition. According to the X-ray powder diffraction (XRPD) results of the isolated solids, three hydrochloride crystal forms (hydrochloride crystal form A/B/C) and free state crystal form A were found, which were characterized by thermogravimetric analysis (TGA) and differential scanning calorimetry (DSC). The molar ratios were determined by ion chromatography (IC).

### Example 14.1: Solubility test of hydrochloride crystal form B

A preliminary solubility test of hydrochloride crystal form B starting material sample was carried out using 20 single solvents and 6 mixed solvents at room temperature (RT, 25±3 °C), including the following steps: about 2 mg of the starting material sample was weighed into a 3-mL vial, and then the corresponding solvent was gradually added until the solid was dissolved clearly; if the sample was still not dissolved clearly after adding 2 mL of the solvent, no more solvent was added. The solubility range in the corresponding solvent was calculated based on the mass of the sample and the volume of the added solvent, as shown in Table 35. The data were used as a base to guide the selection of solvent in the screening tests.

**Table 35 Preliminary solubility of crystal form B of the hydrochloride at room temperature**

| Solvent (v/v) | Solubility (mg/mL) | Solvent (v/v) | Solubility (mg/mL) |
|---|---|---|---|
| MeOH | S>36.0 | 1,4-Dioxane | S<1.0 |
| EtOH | 10.0<S<20.0 | ACN | S<1.1 |
| IPA | S<1.1 | CHCl₃ | S<1.1 |
| Acetone | S<1.0 | DCM | S<1.0 |
| MIBK | S<1.0 | n-Heptane | S<1.0 |
| EtOAc | S<1.0 | Toluene | S<1.0 |
| IPAc | S<1.0 | DMAc | S>40.0 |
| MTBE | S<1.0 | DMSO | S>42.0 |
| THF | S<1.1 | NMP | S>42.0 |
| 2-MeTHF | S<1.0 | H₂O | S<1.0 |
| ACN/H₂O (1:1) | S>40.0 | THF/H₂O (1:1) | 20.0<S<40.0 |
| MeOH/H₂O (1:1) | 11.0<S<22.0 | IPA/H₂O (1:1) | S>40.0 |
| EtOH/H₂O (1:1) | 22.0<S<44.0 | DMF/H₂O (1:1) | 22.0<S<44.0 |

### Example 14.2: Polymorph screening

Crystal form B of a hydrochloride of compound A was used as a starting material, and a total of 50 polymorph screening tests were set up using screening methods including, slow evaporation, temperature cycling with stirring, grinding, suspension and stirring at room temperature and 50 °C, and antisolvent addition. A total of three hydrochloride crystal forms (hydrochloride crystal form A/B/C) and free state crystal form A were found. The specific test methods and results are summarized in Table 36.

**Table 36 Summary of the polymorph screening test results**

| **Test method** | **Number of tests** | **Result** |
|---|---|---|
| Slow evaporation | 3 | Hydrochloride crystal form A/amorphous |
| Temperature cycling with stirring | 9 | Hydrochloride crystal form A/B/free state crystal form A |
| Grinding | 2 | Hydrochloride crystal form B/A+B |
| Suspension and stirring at room temperature | 14 | Hydrochloride crystal form A/B/free state crystal form A |
| Suspension and stirring at 50°C | 8 | Hydrochloride crystal form A/B/free state crystal form A |
| Antisolvent addition | 14 | Hydrochloride crystal form A/C/free state crystal form A/amorphous |
| In total | 50 | Hydrochloride crystal form A/B/C/free state crystal form A/amorphous |

### Example 14.2.1: Slow evaporation

A total of 3 slow evaporation tests were set up using different solvent systems. About 20 mg of the starting material was weighed into a 3-mL vial, and 1.0 to 2.0 mL of a solvent listed in Table 37 was added to dissolve the sample (a 0.45 µm PTFE filter was used to filter the undissolved sample). The vial was sealed with a sealing film, and 4 pinholes were punched in the sealing film. The sample was placed at room temperature to evaporate slowly. The resulting solid was collected and subjected to XRPD analysis. The test results are shown in Table 37. A hydrochloride crystal form A and amorphous products were obtained.

**Table 37 Summary of the slow evaporation test**

| **Test No.** | **Solvent (v/v)** | **Result** |
|---|---|---|
| A1 | EtOH | Hydrochloride crystal form A |
| A2 | THF/H₂O (1:1) | Amorphous |
| A3 | ACN/H₂O (1:1) | Amorphous |

### Example 14.2.2: Temperature cycling with stirring

A total of 9 tests of temperature cycling with stirring were set up using different solvent systems. About 20 mg of crystal form B of the hydrochloride was weighed into an HPLC glass vial, and 0.5 mL of a solvent listed in Table 38 was added, respectively. The resulting suspension was magnetically stirred (1000 rpm) under temperature cycling (the sample was heated to 50 °C and then cooled to 5 °C at a rate of 0.1 °C/minute. The cycle was then repeated, and finally the sample was maintained at 5 °C), and centrifuged to collect a solid. The solid was subjected to XRPD analysis. The test results are shown in Table 38. A hydrochloride crystal form A/B and free state crystal form A were obtained.

**Table 38 Summary of the test of temperature cycling with stirring**

| **Test No.** | **Solvent (v/v)** | **Result** |
|---|---|---|
| A1 | MeOH/MTBE (1:9) | Hydrochloride crystal form A |
| A2 | IPA | Hydrochloride crystal form A |
| A3 | IPAc | Hydrochloride crystal form A |
| A4 | ACN | Hydrochloride crystal form A |
| A5 | Toluene | Hydrochloride crystal form A |
| A6 | Acetone/H₂O (1:9) | Free state crystal form A |
| A7 | THF | Hydrochloride crystal form B |
| A8 | NMP/H₂O (1:9) | Free state crystal form A |
| A9 | EtOH/n-heptane (1:1) | Hydrochloride crystal form A |

### Example 14.2.3: Grinding

A total of 2 grinding tests were set up under different conditions. About 20 mg of crystal form B of the hydrochloride was weighed into a mortar, and 20 µL of a solvent listed in Table 39 was added, respectively. The sample was grinded manually for 3 to 5 minutes. The resulting solid was collected and subjected to XRPD analysis. The test results are shown in Table 39. A hydrochloride crystal form B and a hydrochloride crystal form A+B were obtained.

**Table 39 Summary of the grinding test**

| **Test No.** | **Solvent** | **Result** |
|---|---|---|
| A1 | N/A | Hydrochloride crystal form B |
| A2 | H₂O | Hydrochloride crystal form A+B |

### Example 14.2.4: Suspension and stirring at room temperature

A total of 14 tests of suspension and stirring at room temperature were set up using different solvent systems. About 20 mg of crystal form B of the hydrochloride was weighed into an HPLC glass vial, and 0.5 mL of a solvent listed in Table 40 was added, respectively. The resulting turbid liquid was magnetically stirred (1000 rpm) at room temperature for about 3 days, and then centrifuged to collect a solid. The solid was subjected to XRPD analysis. The test results are shown in Table 40. A hydrochloride crystal form A/B and free state crystal form A samples were obtained.

**Table 40 Summary of the test of suspension and stirring at room temperature**

| **Test No.** | **Solvent (v/v)** | **Result** |
|---|---|---|
| A1 | MeOH/H₂O (1:9) | Free state crystal form A |
| A2 | IPAc | Hydrochloride crystal form B |
| A3 | MTBE | Hydrochloride crystal form A |
| A4 | MIBK | Hydrochloride crystal form A |
| A5 | DCM | Hydrochloride crystal form A |
| A6 | Toluene | Hydrochloride crystal form A |
| A7 | H₂O | Free state crystal form A |
| A8 | ACN | Hydrochloride crystal form A |
| A9 | 2-MeTHF | Hydrochloride crystal form B |
| A10 | DMSO/H₂O (1:9) | Free state crystal form A |
| A11 | IPA/H₂O (982:18, water activity a_{w}~0.2) | Hydrochloride crystal form A |
| A12 | IPA/H₂O (957:43, water activity a_{w}~0.4) | Hydrochloride crystal form A |
| A13 | IPA/H₂O (918:82, water activity a_{w}~0.6) | Hydrochloride crystal form A |
| A14 | IPA/H₂O (849:151, water activity a_{w}~0.8) | Hydrochloride crystal form A* |

| | | |
|---|---|---|
| *: The sample was clear at room temperature, and was clear after being stirred at 5 °C for 3 days. The sample was then transferred to room temperature without sealing and slowly evaporated to give a solid. | | |

### Example 14.2.5: Suspension and stirring at 50 °C

A total of 8 tests of suspension and stirring at 50 °C were set up using different solvent systems. About 20 mg of crystal form B of the hydrochloride was weighed into an HPLC glass vial, and 0.5 mL of a solvent listed in Table 41 was added, respectively. The resulting suspension was magnetically stirred (1000 rpm) at 50 °C for about 3 days, and then centrifuged to collect a solid. The solid was subjected to XRPD analysis. The test results are shown in Table 41. A hydrochloride crystal form A/B and free state crystal form A were obtained.

**Table 41 Summary of the test of suspension and stirring at 50 °C**

| **Test No.** | **Solvent (v/v)** | **Result** |
|---|---|---|
| A1 | EtOH/H₂O (1:9) | Free state crystal form A |
| A2 | Acetone | Hydrochloride crystal form A |
| A3 | EtOAc | Hydrochloride crystal form A |
| A4 | n-Heptane | Hydrochloride crystal form B |
| A5 | H₂O | Free state crystal form A |
| A6 | THF | Hydrochloride crystal form B |
| A7 | 1,4-Dioxane | Hydrochloride crystal form B |
| A8 | CHCl₃ | Hydrochloride crystal form A |

### Example 14.2.6: Antisolvent addition

A total of 14 antisolvent addition tests were set up using different solvents. About 20 mg of the starting material was weighed into a 20-mL vial, and the solid was completely dissolved with 0.8-1.4 mL of solvent (in Table 42) (the undissolved samples were filtered with a 0.45 µm PTFE filter to obtain a clear solution). An antisolvent in Table 42 was added dropwise to the clear solution while stirring (1000 rpm) until a solid precipitated. Or, if there was no solid precipitated when the total volume of the added antisolvent reached 10 mL, the sample was suspended and stirred at 5 °C. The clear samples were transferred to -20 °C for suspension and stirring. If still clear, the samples were transferred to room temperature and evaporated. The precipitated solid was separated and subjected to XRPD analysis. The results are shown in Table 42. A hydrochloride crystal form A/C, free state crystal form A and an amorphous sample were obtained in the antisolvent addition tests.

**Table 42 Summary of the antisolvent addition test**

| **Test No.** | **Solvent (v/v)** | **Antisolvent** | **Result** |
|---|---|---|---|
| A1 | MeOH | H₂O | Free state crystal form A* |
| A2 | MeOH | MTBE | Hydrochloride crystal form A |
| A3 | MeOH | IPA | Hydrochloride crystal form A** |
| A4 | MeOH | Toluene | Hydrochloride crystal form A** |
| A5 | MeOH | EtOAc | Hydrochloride crystal form A |
| A6 | ACN/H₂O (1:1) | H₂O | Free state crystal form A |
| A7 | ACN/H₂O (1:1) | THF | Hydrochloride crystal form C^{#} |
| A8 | ACN/H₂O (1:1) | IPA | Hydrochloride crystal form A^{#} |
| A9 | NMP | H₂O | Free state crystal form A* |
| A10 | NMP | MTBE | Hydrochloride crystal form A |
| A11 | DMSO | H₂O | Amorphous* |
| A12 | DMSO | IPAc | Free state crystal form A** |
| A13 | DMAc | H₂O | Free state crystal form A* |
| A14 | DMAc | Toluene | Hydrochloride crystal form A** |

| | | | |
|---|---|---|---|
| *: The sample formed a gel at room temperature, and formed a gel after being subjected to a temperature cycling (50 °C to 5 °C, 0.1 °C/min) treatment. The sample was transferred to room temperature and slowly evaporated without sealing to give a solid. ^{#}: The sample was clear at room temperature, and was clear after being stirred at 5 °C for 3 days. The sample was transferred to room temperature and slowly evaporated without sealing to give a solid. **: The sample was clear at room temperature, and was clear after being stirred at 5 °C for 3 days. The sample was clear after being stirred at -20 °C for 1 day, and was transferred to room temperature and slowly evaporated without sealing to give a solid. | | | |

### Example 14.3: Hydrochloride crystal form B

### XRPD analysis

Fig. 22 shows the XRPD pattern of crystal form B of the hydrochloride of compound A, in which the diffraction angle 2θ°, d-spacing and relative intensity of the main peaks are listed in Table 43.

**Table 43 XRPD peak search data of crystal form B of the hydrochloride**

| **Diffraction angle [°2θ]** | **D-spacing [Å]** | **Relative intensity [%]** |
|---|---|---|
| 4.4605 | 19.81 | 66.94 |
| 8.7286 | 10.13 | 26.07 |
| 11.0927 | 7.98 | 38.57 |
| 13.6554 | 6.48 | 21.81 |
| 14.3530 | 6.17 | 27.70 |
| 15.0742 | 5.88 | 28.45 |
| 16.4625 | 5.38 | 18.96 |
| 17.4392 | 5.09 | 18.87 |
| 18.1261 | 4.89 | 70.38 |
| 18.8298 | 4.71 | 17.61 |
| 21.3539 | 4.16 | 100.00 |
| 21.6675 | 4.10 | 44.82 |
| 22.1895 | 4.01 | 14.09 |
| 23.1699 | 3.84 | 21.40 |
| 24.3628 | 3.65 | 13.40 |
| 25.4251 | 3.50 | 7.40 |
| 25.9553 | 3.43 | 14.71 |
| 26.7822 | 3.33 | 13.92 |
| 27.4670 | 3.25 | 11.35 |
| 28.3711 | 3.15 | 10.41 |
| 29.4979 | 3.03 | 6.54 |
| 30.3649 | 2.94 | 5.76 |
| 31.3462 | 2.85 | 8.72 |
| 32.3419 | 2.77 | 7.22 |
| 34.9485 | 2.57 | 6.70 |

### DSC and TGA

The TGA/DSC results (Fig. 23) show that the sample has a weight loss of 1.32% when heated to 70 °C, and a weight loss of 5.32% at 70 to 150 °C, and has 3 endothermic peaks at 123.1 °C, 135.2 °C, and 187.5 °C (peak temperature). The crystalline form of crystal form B of the hydrochloride does not change after being heated to 90 °C, and crystal form B of the hydrochloride has a small weight loss in TGA from room temperature to 70 °C, so crystal form B of the hydrochloride is identified to be an anhydrous crystal form.

### IC

The IC results show that the chloride ion content in the sample after being heated to 150 °C is 3.52wt%, and the corresponding acid-base molar ratio is calculated to be 0.6. Therefore, it is inferred that the weight loss in TGA corresponds to a deacidification signal.

### Example 14.4: Hydrochloride crystal form C

### XRPD analysis

Fig. 24 shows the XRPD pattern of crystal form C of the hydrochloride of compound A, in which the diffraction angle 2θ°, d-spacing and relative intensity of the main peaks are listed in Table 44.

**Table 44 XRPD peak search data of crystal form C of the hydrochloride**

| **Diffraction angle [°2θ]** | **D-spacing [Å]** | **Relative intensity [%]** |
|---|---|---|
| 4.1087 | 21.51 | 24.81 |
| 8.1552 | 10.84 | 78.55 |
| 8.7817 | 10.07 | 28.79 |
| 10.6086 | 8.34 | 100.00 |
| 12.2389 | 7.23 | 32.40 |
| 12.5236 | 7.07 | 17.55 |
| 13.4135 | 6.60 | 71.00 |
| 15.0471 | 5.89 | 16.21 |
| 16.7792 | 5.28 | 48.77 |
| 17.7190 | 5.01 | 57.72 |
| 19.0603 | 4.66 | 8.76 |
| 20.2194 | 4.39 | 60.20 |
| 21.5208 | 4.13 | 18.11 |
| 24.6386 | 3.61 | 19.74 |
| 26.6633 | 3.34 | 20.56 |
| 27.7318 | 3.22 | 21.71 |

### DSC and TGA

The TGA/DSC results (Fig. 25) show that the sample has a weight loss of 5.53% when heated to 80 °C, and a weight loss of 6.72% when further heated to 150 °C, and has 3 endothermic peaks at 60.7 °C, 114.4 °C, and 182.4 °C (peak temperature).

### IC

The IC results show that the acid-base molar ratio of the sample is 1.1.

In order to study the weight loss of crystal form C of the hydrochloride in TGA, a heating test was conducted. The XRPD results show that the crystal form C of the hydrochloride changes when heated to 80 °C and cooled to room temperature, and a diffraction peak of crystal form A of the hydrochloride appears. The sample of crystal form C of the hydrochloride melts when heated to 150 °C. The IC results show that the chloride ion content in the sample after being heated to 150 °C is 2.69 wt%, and the corresponding acid-base molar ratio is calculated to be 0.4. Therefore, it is inferred that the weight loss in TGA corresponds to a deacidification signal.

### Example 15: Dynamic vapour sorption test

A dynamic vapour sorption (DVS) test was conducted on crystal form A of the free state and crystal form B of the hydrochloride.

A certain amount of powder was taken and put into a sample pan, and hygroscopicity of the sample was evaluated with DVS according to the following instrument method:

| | |
|---|---|
| Temperature (°C) | 25 |
| N₂ flow rate (ml/min) | 200 |
| dm/dt (%/min) | 0.002 |
| Minimum equilibrium time (min) | 10 |
| Maximum equilibrium time (min) | 180 |
| Relative humidity range (%) | Humidity range: 50%-95%-0%-95%-50% |
| | Increase or decrease: 10% (90%-0%-90%), 5% (90%-95%-90%) |

The DVS test results are shown in Table 45.

**Table 45**

| **Crystal form type** | **Water sorption capacity at 80%RH, %** | **Water sorption capacity at 90%RH, %** | **Water sorption capacity at 95%RH, %** |
|---|---|---|---|
| Free state crystal form A | 0.3556 | 0.4782 | 0.6525 |
| Hydrochloride crystal form B | 0.455 | 0.625 | 1.046 |

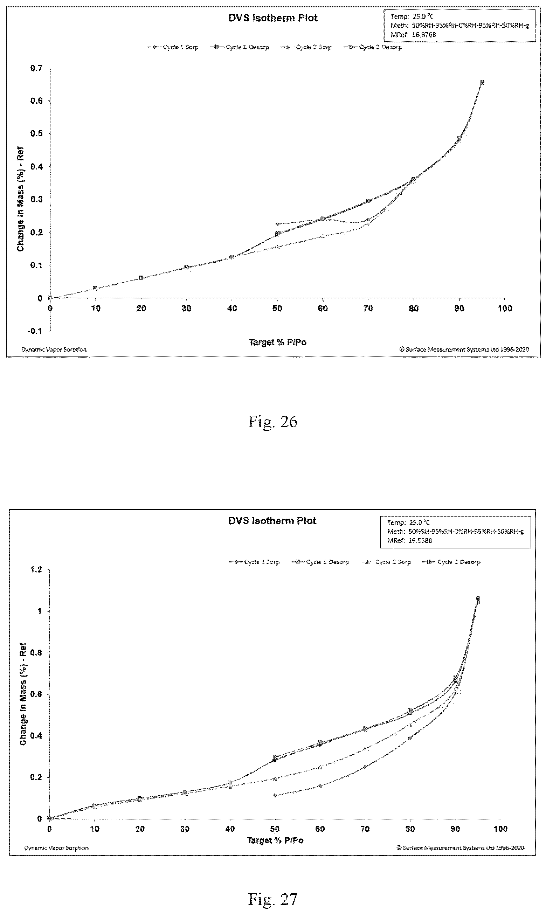

It can be seen from Table 45, Fig. 26 and Fig. 27 that crystal form A of the free state has a moisture-induced weight gain of less than 0.5% at a relative humidity of less than or equal to 90%, and a moisture-induced weight gain of less than 0.7% at a relative humidity of 95%; crystal form B of the hydrochloride has a moisture-induced weight gain of less than 0.7% at a relative humidity of less than or equal to 90%.

Therefore, crystal form A of the free state and crystal form B of the hydrochloride have advantages in hygroscopicity, are suitable for drug development, and can meet the pharmaceutical requirements for production, transportation and storage.

### Example 16: Dissolution stability of free state crystal form A in different pH and biologically relevant media

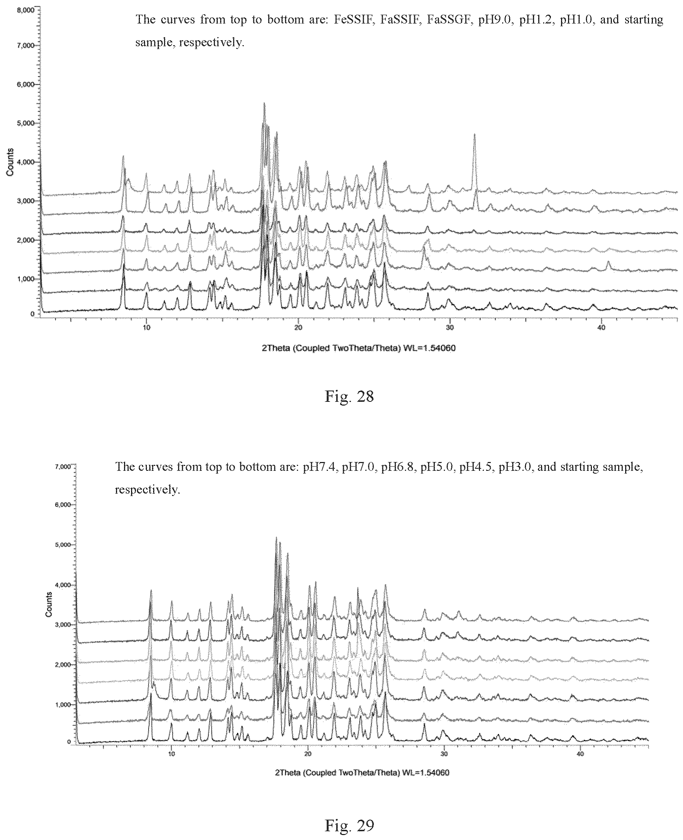

The solubility of crystal form A of the free state under the conditions of pH 1.0, 1.2, 3.0, 4.5, 5.0, 6.8, 7.0, 7.4, and 9.0, as well as FaSSGF (pH 1.6), FaSSIF (pH 6.5), and FeSSIF (pH 5.0) was tested. And XRPD was performed before and after the test, as shown in Fig. 28 and Fig. 29. The results show that the crystal form A of the free state does not change under all the above test conditions, so the crystal form A of the free state is stable.

### Example 17: Forced degradation stability test of free state crystal form A (under conditions of heat, humidity and heat, and light)

Crystal form A of the free state was placed under the following different conditions for the forced degradation stability test:
About 10 mg of the sample powder was weighed into a vial and placed under the conditions of 50 °C, 80°C, and 40 °C/75%RH (unsealed) for 1 day, 2 weeks, and 4 weeks, respectively. And then, the thermal stability was evaluated.
About 10 mg of the sample powder was weighed and placed in a transparent vial, an amber vial, and a transparent vial sealed with aluminum foil, respectively. Those vials were placed in a light box for 10 days, with a total illumination of 1.2×10⁶ Lux·hr and a near-UV energy not less than 200w·hr/m², for photostability evaluation.

The samples were taken at each time point for HPLC and XRPD analysis to evaluate the chemical and physical stability.

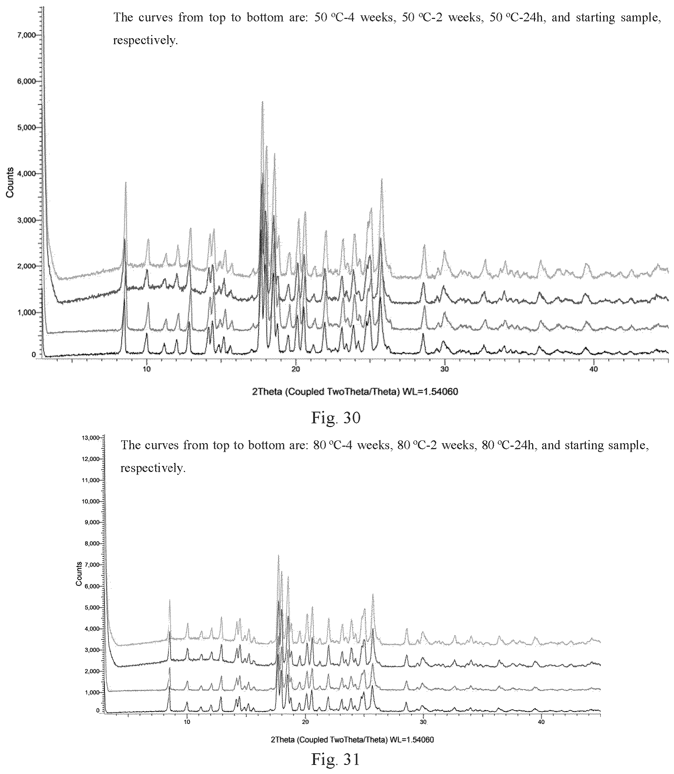

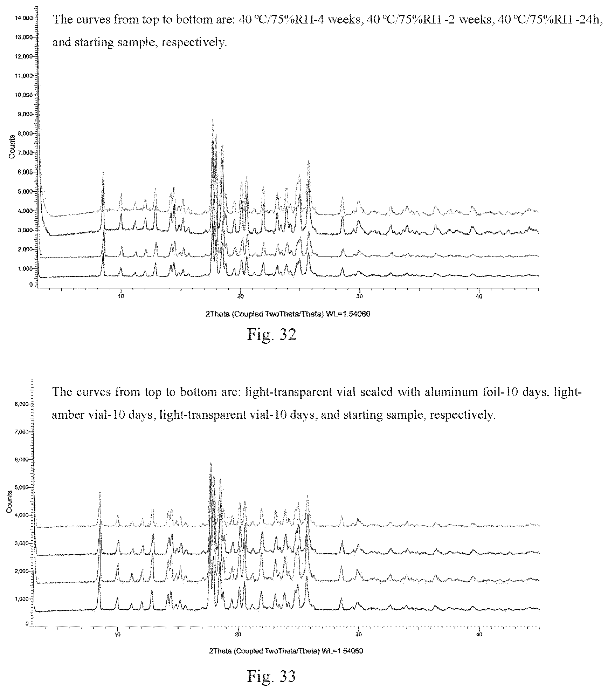

The graphs showing the comparison of XRPD patterns before and after the stability tests are as shown in Fig. 30-33.

| **Test conditions** | **Time point** | **Purity, %** |
|---|---|---|
| Starting | 0h | 99.10 |
| 50°C | 24 hours | 99.08 |
| | 2 weeks | 99.10 |
| | 4 weeks | 99.11 |
| 80°C | 24 hours | 99.12 |
| | 2 weeks | 99.12 |
| | 4 weeks | 99.14 |
| 40°C/75%RH | 24 hours | 99.09 |
| | 2 weeks | 99.15 |
| | 4 weeks | 99.15 |
| Light-transparent vial | 10 days | 99.10 |
| Light-amber vial | 10 days | 99.17 |
| Light-transparent vial sealed with aluminum foil | 10 days | 99.17 |

It can be seen from the above table that, the crystalline form of crystal form A of the free state does not change and has a high purity under the above test conditions, indicating that crystal form A of the free state has excellent high-temperature and humidity-thermal stability. At the same time, light has no effect on the stability of crystal form A of the free state. Therefore, crystal form A of the free state has excellent forced degradation stability.

### Example 18: High-temperature, high-humidity stability, and photostability test of free state crystal form A

### HPLC conditions

| Column | Agilent Poroshell 120 PheHex;4.6*150mm*2.7um | |
|---|---|---|
| Mobile phase A | 5mm NH₄H₂PO₄ in H₂O (pH 4.58) | |
| Mobile phase B | ACN | |
| Wavelength | 220nm | |
| Column temperature (°C) | 40 | |
| Injector temperature | 5°C | |

| Diluent | 0.1% H₃PO₄ in (THF: H₂O=1:1) | |
|---|---|---|
| Flow rate (ml/min) | 1.0 | |
| Injection volume (µl) | 5 | |
| Sample concentration (mg/ml) | 0.5 | |

| Table of flow gradient | | |
|---|---|---|
| Time (min) | Mobile phase (%) | Mobile phase (%) |
| 0.00 | 65 | 35 |
| 14.00 | 55 | 45 |
| 18.00 | 15 | 85 |
| 22.00 | 15 | 85 |
| 23.00 | 65 | 35 |
| 28.00 | Stop | |

### (1) High-temperature stability of free state crystal form A

About 300 mg of free state crystal form A raw material was weighed into a petri dish, and placed in a 60°C oven without cover for a high-temperature stability test. The resulting sample was sampled at day 6, day 14 and day 34 for HPLC analysis. The results are shown in the table below.

| Substance | 0h | 60°C | 60°C | 60°C |
|---|---|---|---|---|
| | | Day 6 | Day 14 | Day 34 |
| Purity (Area%) | | | | |
| Free state crystal form A | 98.17 | 99.03 | 99.11 | / |

Conclusion: The sample was still stable after being placed at 60°C for 14 days.

### (2) High-humidity stability of free state crystal form A

About 300 mg of free state crystal form A raw material was weighed into a petri dish, and placed under a condition of 90% RH (saturated KNO₃ solution dryer) without cover, for a high-humidity stability test. The resulting sample was sampled at day 6, day 14 and day 34 for HPLC analysis. The results are shown in the table below.

| Substance | 0 hour | 90%RH | 90%RH | 90%RH |
|---|---|---|---|---|
| | | Day 6 | Day 14 | Day 34 |
| Purity (Area%) | | | | |
| Free state crystal form A | 98.17 | 98.99 | 99.09 | / |

Conclusion: The sample was still stable after being placed under the condition of 90% relative humidity for 14 days.

### (3) Photostability of free state crystal form A

About 300 mg of free state crystal form A raw material was weighed into a petri dish, and placed under direct sunlight for a photostability test. The resulting sample was sampled at day 4 and day 15 for HPLC analysis. The results are shown in the table below.

| Peak name | 0 hour | Photostability Day 4 | Photostability Day 15 |
|---|---|---|---|
| Purity (Area%) | | | |
| Free state crystal form A | 98.17 | 99.07 | 99.10 |

Conclusion: The sample was still stable after being placed under sunlight for 15 days.

### Example 19: High-temperature and high-humidity stability test of hydrochloride crystal form B

About 10 mg of hydrochloride crystal form B raw material was weighed into a petri dish and placed under the conditions shown in the table below for high-temperature and high-humidity stability tests.

| Substance | RT (min) | Starting point | 60°C | | | 80°C | | |
|---|---|---|---|---|---|---|---|---|
| | | | Day 2 | Day 5 | Day 9 | Day 2 | Day 5 | Day 9 |
| | | Purity (Area%) | | | | | | |
| Hydrochloride crystal form B | 13.045 | 99.72 | 99.55 | 99.43 | 99.55 | 95.20 | 94.65 | 96.80 |

| Substance | RT (min) | Starting point | 25 °C/75%RH | | | 40°C/75%RH | | |
|---|---|---|---|---|---|---|---|---|
| | | | Day 2 | Day 5 | Day 9 | Day 2 | Day 5 | Day 9 |
| | | Purity (Area%) | | | | | | |
| Hydrochloride crystal form B | 13.045 | 99.72 | 99.72 | 99.56 | 99.64 | 99.67 | 99.38 | 99.33 |

Conclusion: Crystal form B of the hydrochloride was not as stable as crystal form A of the free state under the high-temperature and high-humidity conditions.

The above is a further detailed description of the present disclosure in connection with the specific alternative embodiments, and the specific embodiments of the present disclosure are not limited to the description. For those skilled in the art, without departing from the concept of the present disclosure, some simple deductions or substitutions can be made, which should be regarded as falling within the protection scope of the present disclosure.

## Claims

1. A crystal form A of a compound of formula (A): 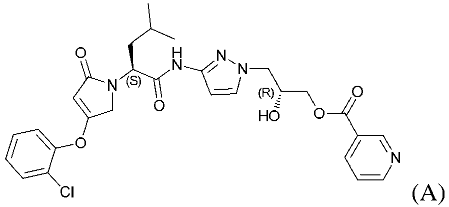 which is **characterized by** an X-ray powder diffraction pattern obtained using CuKα radiation including at least the characteristic peaks represented by the following °2θ: 8.5±0.2, 17.7±0.2, 18.0±0.2, and 18.6±0.2.

2. The crystal form A of the compound of formula (A) according to claim 1, which is **characterized by** an X-ray powder diffraction pattern obtained using CuKα radiation further including the characteristic peaks represented by the following °2θ: 10.1±0.2, 14.5±0.2, 20.2±0.2, 20.6±0.2, 25.1±0.2, and 25.7±0.2.

3. The crystal form A of the compound of formula (A) according to claim 1 or 2, which is **characterized by** an X-ray powder diffraction pattern obtained using CuKα radiation further including the characteristic peaks represented by the following °2θ: 12.9±0.2, 15.2±0.2, 22.0±0.2, 23.2±0.2, and 23.9±0.2.

4. The crystal form A of the compound of formula (A) according to any one of claims 1 to 3, which is **characterized by** an X-ray powder diffraction pattern obtained using CuKα radiation further including the characteristic peaks represented by the following °2θ: 11.2±0.2, 12.1±0.2, 14.2±0.2, 15.7±0.2, 19.6±0.2, 21.2±0.2, 28.6±0.2, and 30.0±0.2.

5. The crystal form A of the compound of formula (A) according to claim 1, which is **characterized by** an X-ray powder diffraction pattern obtained using CuKα radiation including the following characteristic peaks:
| Angle °2θ±0.2 °2θ | Relative intensity % |
|---|---|
| 8.54 | 97.6 |
| 10.05 | 36.1 |
| 11.23 | 13.8 |
| 12.10 | 20.5 |
| 12.91 | 25.9 |
| 14.19 | 28.0 |
| 14.48 | 36.3 |
| 15.25 | 21.7 |
| 15.67 | 7.4 |
| 17.72 | 97.9 |
| 18.02 | 100.0 |
| 18.55 | 78.1 |
| 19.55 | 12.9 |
| 20.17 | 37.3 |
| 20.60 | 45.3 |
| 21.24 | 5.8 |
| 22.01 | 25.1 |
| 23.16 | 19.0 |
| 23.94 | 21.6 |
| 25.09 | 30.8 |
| 25.74 | 33.7 |
| 28.62 | 9.2 |
| 29.98 | 6.6 |

6. The crystal form A of the compound of formula (A) according to claim 1, **characterized in that** it has an X-ray powder diffraction pattern substantially as shown in Fig. 7.

7. The crystal form A of the compound of formula (A) according to any one of claims 1 to 6, **characterized in that** it has an endothermic peak at 135.5±2 °C in differential scanning calorimetry analysis.

8. The crystal form A of the compound of formula (A) according to any one of claims 1 to 7, **characterized in that** it has a weight loss of 1.36±0.5% at 150°C in thermogravimetric analysis.

9. Crystal form A of a hydrochloride of a compound of formula (A): 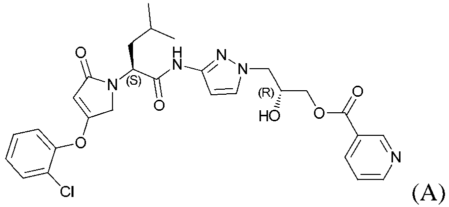 which is **characterized by** an X-ray powder diffraction pattern obtained using CuKα radiation including at least the characteristic peaks represented by the following °2θ: 4.1±0.2, 15.3±0.2, and 18.5±0.2.

10. Crystal form A of the hydrochloride of the compound of formula (A) according to claim 9, which is **characterized by** an X-ray powder diffraction pattern obtained using CuKα radiation further including the characteristic peaks represented by the following °2θ: 8.1±0.2, 10.6±0.2, 11.9±0.2, 22.1±0.2, and 22.9±0.2.

11. Crystal form A of the hydrochloride of the compound of formula (A) according to claim 9 or 10, which is **characterized by** an X-ray powder diffraction pattern obtained using CuKα radiation further including the characteristic peaks represented by the following °2θ: 12.2±0.2, 17.9±0.2, 21.5±0.2, and 24.9±0.2.

12. Crystal form A of the hydrochloride of the compound of formula (A) according to claim 9, which is **characterized by** an X-ray powder diffraction pattern obtained using CuKα radiation including the following characteristic peaks:
| Angle °2θ±0.2 °2θ | Relative intensity % |
|---|---|
| 4.06 | 86.1 |
| 8.11 | 30.3 |
| 10.61 | 36.7 |
| 11.89 | 39.2 |
| 12.18 | 23.0 |
| 15.27 | 66.8 |
| 15.66 | 19.5 |
| 16.28 | 12.1 |
| 16.68 | 20.0 |
| 17.89 | 29.9 |
| 18.45 | 100.0 |
| 19.45 | 12.6 |
| 20.41 | 15.7 |
| 21.51 | 23.7 |
| 22.14 | 32.6 |
| 22.94 | 32.0 |
| 23.75 | 5.8 |
| 24.94 | 26.0 |
| 26.89 | 21.1 |
| 27.53 | 11.9 |
| 28.23 | 9.8 |
| 30.64 | 6.5 . |

13. Crystal form A of the hydrochloride of the compound of formula (A) according to claim 9, **characterized in that** it has an X-ray powder diffraction pattern substantially as shown in Fig. 9.

14. Crystal form A of the hydrochloride of the compound of formula (A) according to any one of claims 9 to 13, **characterized in that** it has endothermic peaks at 125.9±2 °C and 177.8±2°C in differential scanning calorimetry analysis.

15. Crystal form A of the hydrochloride of the compound of formula (A) according to any one of claims 9 to 14, **characterized in that** it has a weight loss of 4.91±0.5% at 150°C in thermogravimetric analysis.

16. Crystal form B of a hydrochloride of a compound of formula (A): 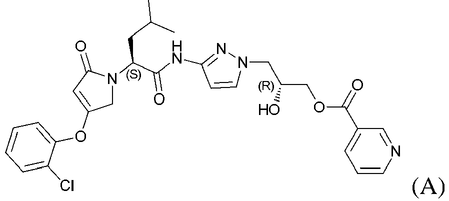 which is **characterized by** an X-ray powder diffraction pattern obtained using CuKα radiation including at least the characteristic peaks represented by the following °2θ: 4.5±0.2, 18.1±0.2, and 21.4±0.2.

17. Crystal form B of the hydrochloride of the compound of formula (A) according to claim 16, which is **characterized by** an X-ray powder diffraction pattern obtained using CuKα radiation further including the characteristic peaks represented by the following °2θ: 11.1±0.2 and 21.7+0.2.

18. Crystal form B of the hydrochloride of the compound of formula (A) according to claim 16 or 17, which is **characterized by** an X-ray powder diffraction pattern obtained using CuKα radiation further including the characteristic peaks represented by the following °2θ: 8.7±0.2, 13.7±0.2, 14.4±0.2, 15.1±0.2, and 23.2±0.2.

19. Crystal form B of the hydrochloride of the compound of formula (A) according to claim 16, which is **characterized by** an X-ray powder diffraction pattern obtained using CuKα radiation including the following characteristic peaks:
| Angle °2θ±0.2 °2θ | Relative intensity % |
|---|---|
| 4.46 | 66.9 |
| 8.73 | 26.1 |
| 11.09 | 38.6 |
| 13.66 | 21.8 |
| 14.35 | 27.7 |
| 15.07 | 28.4 |
| 16.46 | 19.0 |
| 17.44 | 18.9 |
| 18.13 | 70.4 |
| 18.83 | 17.6 |
| 21.35 | 100.0 |
| 21.67 | 44.8 |
| 22.19 | 14.1 |
| 23.17 | 21.4 |
| 24.36 | 13.4 |
| 25.43 | 7.4 |
| 25.96 | 14.7 |
| 26.78 | 13.9 |
| 27.47 | 11.4 |
| 28.37 | 10.4 |
| 29.50 | 6.5 |
| 30.36 | 5.8 |
| 31.35 | 8.7 |
| 32.34 | 7.2 |
| 34.95 | 6.7 |

20. Crystal form B of the hydrochloride of the compound of formula (A) according to claim 16, **characterized in that** it has an X-ray powder diffraction pattern substantially as shown in Fig. 22.

21. Crystal form B of the hydrochloride of the compound of formula (A) according to any one of claims 16 to 20, **characterized in that** it has endothermic peaks at 123.1±2 °C, 135.2±2 °C, and 187.5±2 °C in differential scanning calorimetry analysis.

22. Crystal form B of the hydrochloride of the compound of formula (A) according to any one of claims 16 to 21, **characterized in that** it has a weight loss of 1.32±0.5% at 70°C, and a weight loss of 5.32±0.5% at 70 to 150°C in thermogravimetric analysis.

23. Crystal form C of a hydrochloride of a compound of formula (A): 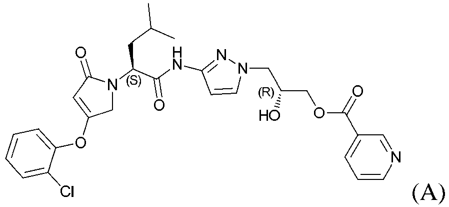 which is **characterized by** an X-ray powder diffraction pattern obtained using CuKα radiation including at least the characteristic peaks represented by the following °2θ: 8.2±0.2, 10.6±0.2, 13.4±0.2, and 20.2±0.2.

24. Crystal form C of the hydrochloride of the compound of formula (A) according to claim 23, which is **characterized by** an X-ray powder diffraction pattern obtained using CuKα radiation further including the characteristic peaks represented by the following °2θ: 16.8±0.2 and 17.7±0.2.

25. Crystal form C of the hydrochloride of the compound of formula (A) according to claim 23 or 24, which is **characterized by** an X-ray powder diffraction pattern obtained using CuKα radiation further including the characteristic peaks represented by the following °2θ: 4.1±0.2, 8.8±0.2, and 12.2±0.2.

26. Crystal form C of the hydrochloride of the compound of formula (A) according to claim 23, which is **characterized by** an X-ray powder diffraction pattern obtained using CuKα radiation including the following characteristic peaks:
| Angle °2θ±0.2 °2θ | Relative intensity % |
|---|---|
| 4.11 | 24.8 |
| 8.16 | 78.6 |
| 8.78 | 28.8 |
| 10.61 | 100.0 |
| 12.24 | 32.4 |
| 12.52 | 17.6 |
| 13.41 | 71.0 |
| 15.05 | 16.2 |
| 16.78 | 48.8 |
| 17.72 | 57.7 |
| 19.06 | 8.8 |
| 20.22 | 60.2 |
| 21.52 | 18.1 |
| 24.64 | 19.7 |
| 26.66 | 20.6 |
| 27.73 | 21.7 . |

27. Crystal form C of the hydrochloride of the compound of formula (A) according to claim 23, **characterized in that** it has an X-ray powder diffraction pattern substantially as shown in Fig. 24.

28. Crystal form C of the hydrochloride of the compound of formula (A) according to any one of claims 23 to 27, **characterized in that** it has endothermic peaks at 60.7±2 °C, 114.4±2 °C, and 182.4±2 °C in differential scanning calorimetry analysis.

29. Crystal form C of the hydrochloride of the compound of formula (A) according to any one of claims 23 to 28, **characterized in that** it has a weight loss of 5.53±0.5% at 80°C, and a weight loss of 6.72±0.5% at 80 to 150°C in thermogravimetric analysis.

30. Crystal form A of a maleate of a compound of formula (A): 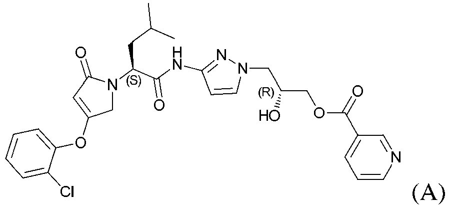 which is **characterized by** an X-ray powder diffraction pattern obtained using CuKα radiation including at least the characteristic peaks represented by the following °2θ: 6.0±0.2 and 18.4±0.2.

31. Crystal form A of the maleate of the compound of formula (A) according to claim 30, which is **characterized by** an X-ray powder diffraction pattern obtained using CuKα radiation further including the characteristic peaks represented by the following °2θ: 10.4±0.2, 15.9±0.2, and 17.4±0.2.

32. Crystal form A of the maleate of the compound of formula (A) according to claim 30 or 31, which is **characterized by** an X-ray powder diffraction pattern obtained using CuKα radiation further including the characteristic peaks represented by the following °2θ: 6.9±0.2, 12.0±0.2, 21.2±0.2, and 21.8±0.2.

33. Crystal form A of the maleate of the compound of formula (A) according to claim 30, which is **characterized by** an X-ray powder diffraction pattern obtained using CuKα radiation including the following characteristic peaks:
| Angle °2θ±0.2 °2θ | Relative intensity % |
|---|---|
| 6.01 | 100.0 |
| 6.92 | 12.6 |
| 10.41 | 18.6 |
| 12.01 | 12.2 |
| 15.93 | 17.4 |
| 17.38 | 30.8 |
| 18.42 | 57.6 |
| 19.46 | 8.1 |
| 21.21 | 12.1 |
| 21.76 | 14.4 |
| 22.93 | 5.1 |
.

34. Crystal form A of the maleate of the compound of formula (A) according to claim 30, **characterized in that** it has an X-ray powder diffraction pattern substantially as shown in Fig. 11.

35. Crystal form B of a maleate of a compound of formula (A): 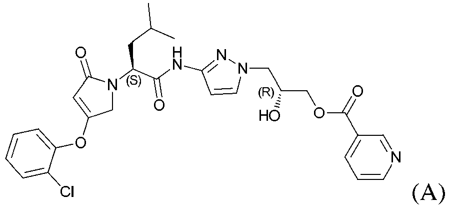 which is **characterized by** an X-ray powder diffraction pattern obtained using CuKα radiation including at least the characteristic peaks represented by the following °2θ: 4.1±0.2, 8.1±0.2, 16.7±0.2, and 20.2±0.2.

36. Crystal form B of the maleate of the compound of formula (A) according to claim 35, which is **characterized by** an X-ray powder diffraction pattern obtained using CuKα radiation further including the characteristic peaks represented by the following °2θ: 17.5±0.2 and 21.3±0.2.

37. Crystal form B of the maleate of the compound of formula (A) according to claim 35 or 36, which is **characterized by** an X-ray powder diffraction pattern obtained using CuKα radiation further including the characteristic peaks represented by the following °2θ: 16.2±0.2, 20.4±0.2, 22.4±0.2, 24.1±0.2, 24.6±0.2, and 26.5±0.2.

38. Crystal form B of the maleate of the compound of formula (A) according to claim 35, which is **characterized by** an X-ray powder diffraction pattern obtained using CuKa radiation including the following characteristic peaks:
| Angle °2θ±0.2 °2θ | Relative intensity% |
|---|---|
| 4.08 | 79.3 |
| 8.13 | 94.1 |
| 10.50 | 21.5 |
| 12.20 | 27.7 |
| 13.31 | 13.4 |
| 15.01 | 21.4 |
| 16.24 | 31.0 |
| 16.68 | 72.3 |
| 17.54 | 60.1 |
| 18.37 | 12.5 |
| 20.17 | 100.0 |
| 20.41 | 34.0 |
| 21.34 | 67.4 |
| 22.40 | 34.6 |
| 22.69 | 26.7 |
| 23.16 | 13.7 |
| 24.14 | 49.6 |
| 24.56 | 30.9 |
| 25.97 | 21.7 |
| 26.55 | 34.4 |
| 27.59 | 20.4 |
| 29.90 | 15.4 |

39. Crystal form B of the maleate of the compound of formula (A) according to claim 35, **characterized in that** it has an X-ray powder diffraction pattern substantially as shown in Fig. 12.

40. Crystal form B of the maleate of the compound of formula (A) according to any one of claims 35 to 39, **characterized in that** it has an endothermic peak at 85.6±2 °C in differential scanning calorimetry analysis.

41. Crystal form B of the maleate of the compound of formula (A) according to any one of claims 35 to 40, **characterized in that** it has a weight loss of 12.02±0.5% at 120 °C in thermogravimetric analysis.

42. Crystal form A of a naphthalene disulfonate of a compound of formula (A): 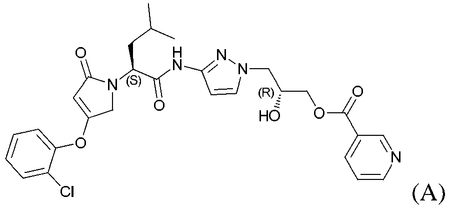 which is **characterized by** an X-ray powder diffraction pattern obtained using CuKα radiation including at least the characteristic peaks represented by the following °2θ: 4.0±0.2 and 13.7±0.2.

43. Crystal form A of the naphthalene disulfonate of the compound of formula (A) according to claim 42, which is **characterized by** an X-ray powder diffraction pattern obtained using CuKa radiation further including the characteristic peaks represented by the following °2θ: 10.3 ±0.2, 15.5±0.2, 21.0±0.2, and 22.3±0.2.

44. Crystal form A of the naphthalene disulfonate of the compound of formula (A) according to claim 42, which is **characterized by** an X-ray powder diffraction pattern obtained using CuKa radiation including the following characteristic peaks:
| Angle °2θ±0.2 °2θ | Relative intensity % |
|---|---|
| 4.03 | 100.0 |
| 10.28 | 31.9 |
| 11.96 | 12.6 |
| 13.68 | 67.6 |
| 15.49 | 28.7 |
| 20.96 | 22.4 |
| 22.27 | 28.0 |
| 26.22 | 15.1 |

45. Crystal form A of the naphthalene disulfonate of the compound of formula (A) according to claim 42, **characterized in that** it has an X-ray powder diffraction pattern substantially as shown in Fig. 14.

46. Crystal form A of the naphthalene disulfonate of the compound of formula (A) according to any one of claims 42 to 45, **characterized in that** it has endothermic peaks at 119.2±2 °C and 207.9±2 °C in differential scanning calorimetry analysis.

47. Crystal form A of the naphthalene disulfonate of the compound of formula (A) according to any one of claims 42 to 46, **characterized in that** it has a weight loss of 5.97±0.5% at 150°C in thermogravimetric analysis.

48. Crystal form A of an oxalate of a compound of formula (A): 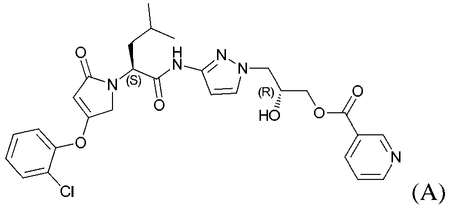 which is **characterized by** an X-ray powder diffraction pattern obtained using CuKα radiation including at least the characteristic peaks represented by the following °2θ: 5.0±0.2 and 15.0±0.2.

49. Crystal form A of the oxalate of the compound of formula (A) according to claim 48, which is **characterized by** an X-ray powder diffraction pattern obtained using CuKα radiation further including the characteristic peaks represented by the following °2θ: 7.4±0.2, 16.5±0.2, 19.3±0.2, 20.1±0.2, 25.2±0.2, and 26.0±0.2.

50. Crystal form A of the oxalate of the compound of formula (A) according to claim 48, which is **characterized by** an X-ray powder diffraction pattern obtained using CuKα radiation including the following characteristic peaks:
| Angle °2θ±0.2 °2θ | Relative intensity % |
|---|---|
| 5.00 | 100.0 |
| 7.37 | 35.6 |
| 15.05 | 60.5 |
| 16.52 | 41.7 |
| 17.39 | 12.7 |
| 18.63 | 8.7 |
| 19.31 | 27.4 |
| 20.10 | 28.4 |
| 20.68 | 13.3 |
| 22.72 | 14.7 |
| 25.21 | 25.8 |
| 26.02 | 21.2 |

51. Crystal form A of the oxalate of the compound of formula (A) according to claim 48, **characterized in that** it has an X-ray powder diffraction pattern substantially as shown in Fig. 16.

52. Crystal form A of the oxalate of the compound of formula (A) according to any one of claims 48 to 51, **characterized in that** it has endothermic peaks at 131.4±2 °C and 192.8±2 °C in differential scanning calorimetry analysis.

53. Crystal form A of the oxalate of the compound of formula (A) according to any one of claims 48 to 52, **characterized in that** it has a weight loss of 3.91±0.5% at 150°C in thermogravimetric analysis.

54. Crystal form A of a hydrobromide of a compound of formula (A): 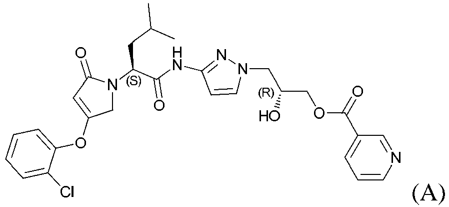 which is **characterized by** an X-ray powder diffraction pattern obtained using CuKα radiation including at least the characteristic peaks represented by the following °2θ: 4.3±0.2 and 8.3±0.2.

55. Crystal form A of the hydrobromide of the compound of formula (A) according to claim 54, which is **characterized by** an X-ray powder diffraction pattern obtained using CuKα radiation further including the characteristic peaks represented by the following °2θ: 12.4±0.2, 17.5±0.2, 17.9±0.2, 20.5±0.2, and 21.3±0.2.

56. Crystal form A of the hydrobromide of the compound of formula (A) according to claim 54, which is **characterized by** an X-ray powder diffraction pattern obtained using CuKα radiation including the following characteristic peaks:
| Angle °2θ±0.2 °2θ | Relative intensity % |
|---|---|
| 4.26 | 42.7 |
| 8.29 | 100.0 |
| 10.63 | 5.4 |
| 12.42 | 29.3 |
| 14.81 | 19.3 |
| 16.53 | 11.4 |
| 16.86 | 19.9 |
| 17.53 | 26.9 |
| 17.90 | 22.6 |
| 20.50 | 32.3 |
| 21.31 | 35.0 |
| 22.52 | 9.6 |
| 22.96 | 9.1 |
| 24.60 | 9.8 |
| 24.96 | 12.8 |
.

57. Crystal form A of the hydrobromide of the compound of formula (A) according to claim 54, **characterized in that** it has an X-ray powder diffraction pattern substantially as shown in Fig. 18.

58. Crystal form B of a hydrobromide of a compound of formula (A): 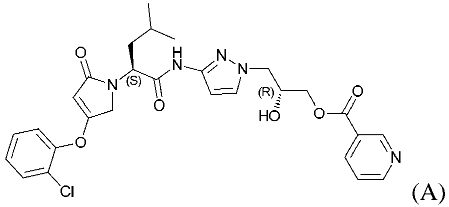 which is **characterized by** an X-ray powder diffraction pattern obtained using CuKα radiation including at least the characteristic peaks represented by the following °2θ: 4.1±0.2, 8.1±0.2, 15.0±0.2, 18.4±0.2, and 24.4±0.2.

59. Crystal form B of the hydrobromide of the compound of formula (A) according to claim 58, which is **characterized by** an X-ray powder diffraction pattern obtained using CuKα radiation further including the characteristic peaks represented by the following °2θ: 17.8±0.2, 20.3±0.2, 21.3±0.2, 22.0±0.2, and 22.8±0.2.

60. Crystal form B of the hydrobromide of the compound of formula (A) according to claim 58, which is **characterized by** an X-ray powder diffraction pattern obtained using CuKα radiation including the following characteristic peaks:
| Angle °2θ±0.2 °2θ | Relative intensity % |
|---|---|
| 4.05 | 100.0 |
| 8.10 | 69.4 |
| 10.57 | 17.3 |
| 12.14 | 19.9 |
| 12.74 | 16.7 |
| 13.54 | 14.6 |
| 15.05 | 74.2 |
| 15.43 | 18.4 |
| 16.23 | 19.8 |
| 17.84 | 27.2 |
| 18.45 | 82.8 |
| 19.27 | 12.0 |
| 20.35 | 37.7 |
| 21.33 | 24.5 |
| 22.02 | 33.6 |
| 22.77 | 32.0 |
| 23.65 | 12.8 |
| 24.44 | 55.0 |
| 26.84 | 14.5 |
| 30.65 | 12.9 |

61. Crystal form B of the hydrobromide of the compound of formula (A) according to claim 58, **characterized in that** it has an X-ray powder diffraction pattern substantially as shown in Fig. 19.

62. Crystal form B of the hydrobromide of the compound of formula (A) according to any one of claims 58 to 61, **characterized in that** it has endothermic peaks at 101.6±2 °C and 113.3±2 °C in differential scanning calorimetry analysis.

63. Crystal form B of the hydrobromide of the compound of formula (A) according to any one of claims 58 to 62, **characterized in that** it has a weight loss of 4.59±0.5% at 90 °C in thermogravimetric analysis.

64. A pharmaceutical composition, comprising the crystal form according to any one of claims 1 to 63 and one or more pharmaceutically acceptable excipients.

65. Use of the crystal form according to any one of claims 1 to 63 or the pharmaceutical composition according to claim 64 in the manufacture of a medicament for the treatment and/or prevention of one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

66. The crystal form according to any one of claims 1 to 63 or the pharmaceutical composition according to claim 64, for use in the treatment and/or prevention of one or more diseases selected from type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

67. A method of treating and/or preventing a disease in a subject, comprising administering to the subject the crystal form according to any one of claims 1 to 63, or the pharmaceutical composition according to claim 64, wherein the disease is selected from one or more of the following: type I diabetes mellitus, type II diabetes mellitus, impaired glucose tolerance, impaired fasting blood glucose, hyperglycemia, postprandial hyperglycemia, overweight, obesity, hypertension, insulin resistance, and metabolic syndrome.

68. Use of the crystal form according to any one of claims 1 to 63 or the pharmaceutical composition according to claim 64 in the manufacture of a medicament for curing diabetes, causing diabetes remission and/or regressing diabetes.

69. The crystal form according to any one of claims 1 to 63 or the pharmaceutical composition according to claim 64, for use in curing diabetes, causing diabetes remission and/or regressing diabetes.

70. A method of curing diabetes, causing diabetes remission and/or regressing diabetes in a subject, comprising administering to the subject the crystal form according to any one of claims 1 to 63, or the pharmaceutical composition according to claim 64.
